(19) Europäisches Patentamt / European Patent Office / Office européen des brevets

(11) **EP 2 877 836 B1**

(12) **FASCICULE DE BREVET EUROPEEN**

(45) Date de publication et mention de la délivrance du brevet:
**21.12.2022 Bulletin 2022/51**

(21) Numéro de dépôt: **13773398.6**

(22) Date de dépôt: **26.07.2013**

(51) Classification Internationale des Brevets (IPC):
**G01N 21/45** *(2006.01)* **B82B 1/00** *(2006.01)*
**G01N 21/55** *(2014.01)* **G01N 21/59** *(2006.01)*
**G01N 21/359** *(2014.01)*

(52) Classification Coopérative des Brevets (CPC):
**G01N 21/359; G01N 21/59**

(86) Numéro de dépôt international:
**PCT/IB2013/056153**

(87) Numéro de publication internationale:
**WO 2014/016813 (30.01.2014 Gazette 2014/05)**

(54) **PROCEDES OPTIQUES POUR L'OBSERVATION D'ECHANTILLONS ET POUR LA DETECTION OU LE DOSAGE D'ESPECES CHIMIQUES OU BIOLOGIQUES**

OPTISCHE VERFAHREN ZUR BEOBACHTUNG VON PROBEN UND ZUM ERFASSEN BZW. MESSEN CHEMISCHER ODER BIOLOGISCHER SPEZIES

OPTICAL METHODS FOR OBSERVING SAMPLES AND FOR DETECTING OR METERING CHEMICAL OR BIOLOGICAL SPECIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.07.2012 FR 1257279**

(43) Date de publication de la demande:
**03.06.2015 Bulletin 2015/23**

(73) Titulaires:
- **Centre National de la Recherche Scientifique (C.N.R.S.)**
  **75016 Paris (FR)**
- **Universite Du Maine (Le Mans)**
  **72085 Le Mans Cédex (FR)**
- **Université d'Aix Marseille**
  **13007 Marseille 7 (FR)**

(72) Inventeurs:
- **AUSSERRÉ, Dominique**
  **F-72370 Soulitré (FR)**
- **ROUSSILLE, Ludovic**
  **F-13006 Marseille (FR)**
- **ZERRAD, Myriam**
  **F-13001 Marseille (FR)**
- **LEMARCHAND, Fabien**
  **F-13480 Cabries (FR)**
- **AMRA, Claude**
  **F-13013 Marseille (FR)**

(74) Mandataire: **Atout PI Laplace**
**Immeuble "Visium"**
**22, avenue Aristide Briand**
**94117 Arcueil Cedex (FR)**

(56) Documents cités:
**WO-A1-2004/001399 FR-A1- 2 818 376**
**FR-A1- 2 872 910**

**Description**

L'invention porte sur un procédé optique pour l'observation d'échantillons, ainsi que sur un procédé optique pour la détection ou le dosage d'espèces chimiques ou biologiques. Ces procédés se basent sur un même principe, nouvellement découvert.

L'invention est susceptible d'être appliquée à différents domaines techniques, tels que la biologie (détection de biomolécules ou microorganismes, observation de cultures cellulaires), les nanotechnologies (visualisation de nano-objets, tels que des nanotubes), la microélectronique, la science des matériaux, etc. Dans certains de ces domaines, elle peut constituer une alternative aux techniques de détection conventionnelles basées sur les plasmons de surfaces, telles que les techniques SPR (de l'anglais « Surface Plasmon Resonance»), LSPR (SPR localisée) et SERS (Spectroscopie Raman Amplifiée par des Surfaces, de l'anglais « Surface-Enhanced Raman Spectroscopy »). Pour une description de ces techniques conventionnelles on peut se rapporter à l'article de M. E. Stewart et al. « Nanostructured Plasmonic Sensors », Chem. Rev. 2008, 108, 494- 521 et à l'article de S. A. Meyer et al « Combining Surface Plasmon Resonance (SPR) Spectroscopy with Surface- Enhanced Raman Scattering (SERS) », Anal. Chem. 2011, 83, 2337-2344.

Une limite de ces techniques est constituée par le fait qu'il est difficile d'obtenir une résolution spatiale élevée qui serait utile, par exemple, pour augmenter la densité d'une biopuce. En effet, ces techniques mettent en œuvre une couche métallique fonctionnalisée, qui doit être éclairée par un faisceau de lumière collimaté, et donc pas focalisé, sous peine d'une perte considérable de contraste (voir l'article précité de S. A. Meyer et al.). Il n'est donc pas possible d'éclairer et observer un capteur plasmonique conventionnel sous microscope optique. Les autres techniques d"imagerie plasmonique, mises en œuvre avec un faisceau d'éclairage parallèle, ont dans tous les cas une résolution limitée à quelques dizaines de microns ; voir par exemple Charles T. Campbell, Gibum Kim « SPR microscopy and its applications to high-throughput analyses of biomolecular binding events and their kinetics » Biomaterials 28 (2007) 2380-2392.

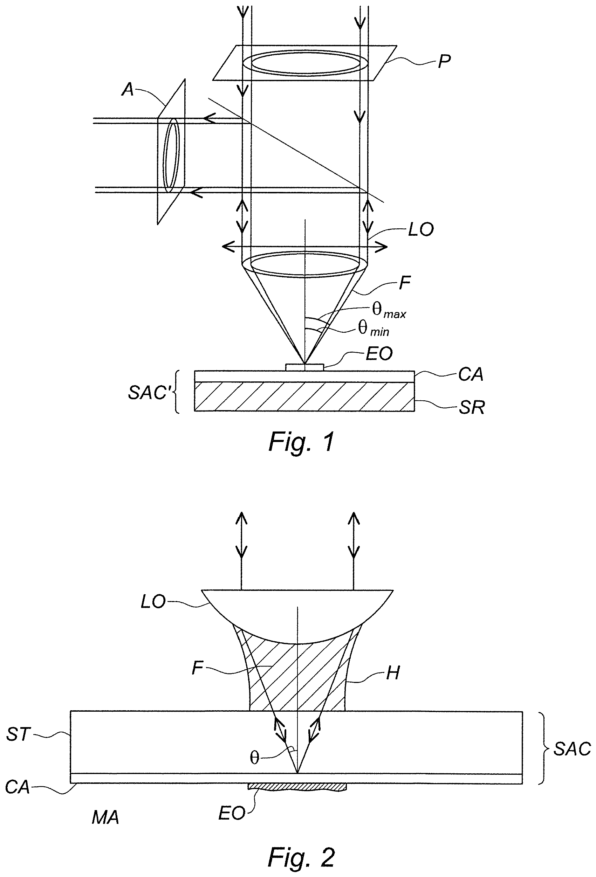

Le document WO 2004/001399 et l'article de O. Ausserré et M. P. Valignat« Surface enhanced ellipsometric contrast (SEEC) basic theory and $\lambda/4$ multilayered solutions», Optics Express, Vol. 15, No. 13, 25 juin 2007, décrivent une technique de microscopie optique à haut contraste, dénommée « contraste ellipsométrique amplifiée par une surface», ou SEEC, qui est illustrée sur la figure 1. Conformément à cette technique, un échantillon à observer EO est déposé sur un support amplificateur de contraste SAC', comprenant un substrat SR, généralement réfléchissant, et une couche ou structure multicouches antireflets CA. L'ensemble constitué par l'échantillon et son support est éclairé par un faisceau F de lumière spatialement incohérente, polarisé linéairement au moyen d'un polariseur P. Le faisceau F peut être annulaire ; en tout cas, il est focalisé sur l'ensemble support-échantillon au moyen d'un objectif LO, et son axe de propagation est perpendiculaire à la surface dudit support. Dans le cas d'un faisceau F annulaire, les rayons de lumière forment avec la normale au support un angle d'incidence compris entre $\theta_{min}$ et $\theta_{max}$; si le faisceau n'est pas annulaire, $\theta_{min}{\rightarrow}0°$. L'observation se fait à travers un analyseur de polarisation A, généralement orienté de telle manière que son axe de polarisation soit perpendiculaire à la direction de polarisation du faisceau F (on dit alors que le polariseur P et l'analyseur A sont croisés) ; avantageusement, l'objectif LO est également utilisé pour l'observation. Ainsi, ledit objectif ainsi que le polariseur et l'analyseur forment un microscope polarisant.

La couche ou structure multicouches antireflets CA est dimensionnée de manière à minimiser - idéalement, à annuler - l'intensité lumineuse qui, réfléchie par le support SAC', traverse l'analyseur A. L'objet, généralement transparent, perturbe la condition d'extinction, et apparaît donc comme une forme lumineuse sur un fond noir.

A proprement parler, la condition d'extinction est satisfaite pour un seul angle d'incidence $\theta_0$, ce qui correspondrait à un éclairage annulaire avec $\theta_{min} = \theta_{max} = \theta_0$ ; mais évidemment dans ces conditions l'intensité lumineuse tend vers zéro. De manière plus réaliste, l'intervalle $\theta_{max} - \theta_{min}$ peut être choisi de l'ordre de 5°. En variante, les documents précités indiquent qu'il est possible d'avoir recours à un éclairage «conique» (c'est-à-dire non annulaire, caractérisé par $\theta_{min} = 0°$), avec $\theta_{max}$ pouvant atteindre 20° voire même 30°. Dans ce cas, le dimensionnement du support SAC' se fait sur la base d'un angle d'incidence moyen.

Dans ces conditions, le choix des conditions d'éclairage relève d'un compromis, qui peut ne pas être pleinement satisfaisant. En effet :

- un éclairage annulaire est difficile à mettre en œuvre et implique une intensité lumineuse d'autant plus faible que l'intervalle $\theta_{max} - \theta_{min}$ est petit ;
- un éclairage conique fortement convergent, avec $\theta_{max}$ de l'ordre de 20° ou plus, conduit à un faible contraste car l'extinction n'est réellement obtenue qu'à un seul angle d'incidence ; et
- un éclairage conique faiblement convergent implique une faible ouverture de l'objectif, ce qui limite la résolution spatiale avec laquelle l'échantillon est observé.

Comme la technique SPR, la méthode SEEC peut être appliquée à des analyses biologiques ou biochimiques. Par exemple, le support SAC' peut constituer le fond d'une boîte de Pétri, auquel cas l'observation doit généralement se faire en immersion dans un milieu de culture, ce qui est parfois malaisé.

Le document WO 02/50513 décrit l'application de la technique SEEC à la réalisation de mesures ellipsométriques avec résolution spatiale, ainsi qu'à la fabrication de capteurs biologiques. Dans ce cas également il est difficile d'obtenir à la fois une résolution spatiale élevée et un contraste satisfaisant; par ailleurs l'observation est parfois malaisée, notamment en ce qui concerne les applications à la biologie ou à la biochimie.

FR2872910 A1 concerne l'observation et l'analyse de caractéristiques nanométriques à l'aide d'un composant optique et un système d'observation en lumière polarisée. Ce document divulgue un composant optique pour l'observation d'un échantillon, comportant un substrat et au moins une couche d'indice complexe et d'épaisseur prédéterminés, destinée à faire apparaître un contraste élevé d'intensité ou de couleur pour des variations de chemin optique, des reliefs, des épaisseurs et des diamètres nanométriques lorsqu'il est observé par réflexion en lumière incohérente convergente autour de l'incidence normale dans des conditions d'extinction de polarisation, la couche d'indice supérieure présentant des propriétés de surface spécifiques lui conférant une affinité sélective par rapport à au moins une caractéristique de l'échantillon.

L'invention vise à remédier aux inconvénients précités de l'art antérieur. Pour ce faire, elle se base sur un effet nouvellement découvert par les présents inventeurs. En effet, ces derniers se sont rendus compte du fait que, lorsque on dépose un objet mince (typiquement une couche d'épaisseur nanométrique) sur un support comportant un substrat transparent et un revêtement métallique ou plus généralement absorbant, puis on l'observe sous microscope en géométrie inversée (c'est-à-dire avec éclairage et observation à travers le substrat), on obtient - pour certaines valeurs de l'épaisseur du revêtement - un contraste relativement élevé et pratiquement indépendant de l'ouverture numérique du microscope dans une large plage de variation de cette dernière. De manière tout aussi surprenante, cet effet est constaté aussi bien en lumière polarisée (observation à travers un polariseur et un analyseur croisés) qu'en lumière non polarisée. Bien que l'utilisation de lumière polarisée permette, dans certaines conditions, une augmentation du contraste, l'utilisation de lumière non polarisée évite la perte de lumière provoquée par le polariseur

et l'analyseur, simplifie la mise en œuvre du procédé et permet d'obtenir des images plus homogènes.

Un objet de l'invention est donc un procédé d'observation d'un échantillon selon la revendication 1.

Un autre objet de l'invention est un procédé de détection ou dosage d'au moins une espèce chimique ou biologique selon la revendication 9.

On rappelle qu'un thalweg est défini comme la ligne de fond d'une vallée.

D'autres caractéristiques, détails et avantages de l'invention ressortiront à la lecture de la description faite en référence aux dessins annexés donnés à titre d'exemple et qui représentent, respectivement :

- La figure 1, décrite ci-dessus, illustre la technique SEEC connue de l'art antérieur ;
- La figure 2 montre un montage optique pour la mise en œuvre d'un procédé d'observation d'un échantillon selon un mode de réalisation de l'invention ;
- La figure 3 illustre la ligne d'inversion de contraste dans le plan n-K d'un support d'échantillon convenant à la mise en œuvre de l'invention (n étant la partie réelle de l'indice de réfraction de la couche amplificatrice de contraste et K sa partie imaginaire) ;
- Les figures 4-1 à 4-56 montrent des surfaces exprimant le contraste avec lequel est observé un échantillon de référence en fonction du demi-angle du cône d'éclairage, e, et de l'épaisseur de la couche amplificatrice de contraste, normalisée par rapport à la longueur d'onde, $e/\lambda$ (dans le cas d'un éclairage polychromatique, $\lambda$ est la longueur d'onde moyenne) ; chaque figure correspond à un matériau différent, caractérisé par un couple $(n,K)$;
- La figure 5 situe les matériaux des figures 4-1 à 4-56 dans le plan n-$\kappa$;
- La figure 6 situe une couche d'or dans le plan n-$\kappa$, à diverses longueurs d'onde d'éclairage ;
- La figure 7 représente une couche amplificatrice de contraste présentant un gradient d'épaisseur;
- La figure 8 représente une couche amplificatrice de contraste fonctionnalisée ;
- Les figures 9 - 12 illustrent respectivement le premier, deuxième, troisième et quatrième mode de réalisation d'un procédé de détection ou dosage d'au moins une espèce chimique ou biologique selon l'invention.

Une couche anti-réfléchissante conventionnelle présente une épaisseur égale à $\lambda/4n_1$ ($\lambda$ étant la longueur d'onde de la lumière dans le vide et $n_1$ l'indice de réfraction de la couche, supposé réel) et un indice de réfraction donné par:

$$n_1 = \sqrt{n_0 n_2} \qquad\qquad (1)$$

où $n_2$ est l'indice de réfraction du substrat sur lequel la couche est déposée et $n_0$ l'indice de réfraction du milieu qui se trouve au-dessus de la couche (par exemple, l'air). Les indices $n_0$ et $n_2$ sont considérés réels, tout comme n1.

Un substrat sur lequel est déposée une couche anti-réfléchissante peut être utilisée comme support amplificateur de contraste, mais il s'avère mal adapté à l'observation d'objets de faible contraste sous microscope optique. La technique SEEC modifie la condition de suppression de la réflexion pour l'adapter au cas d'une observation en lumière polarisée et à travers un analyseur de polarisation, mais elle ne permet de surmonter cette limitation que d'une manière très partielle, comme cela a été discuté plus haut.

Afin d'obtenir une amplification de contraste satisfaisante dans une très large plage d'angles d'incidence de la lumière d'éclairage (et donc, notamment, sous microscope à grande ouverture numérique) la présente invention propose d'exploiter un degré de liberté additionnel : la partie imaginaire $\kappa$ de l'indice de réfraction, ce qui implique l'utilisation d'une couche absorbante ou métallique.

On considère le cas, illustré sur la figure 2, d'une couche mince

CA ayant un indice de réfraction $\tilde{n}_1 = n_1 - j\kappa_1$ dont la composante imaginaire est non négligeable, par exemple supérieure à 0,001 (on suit la convention selon laquelle $\kappa_1$ positif correspond à une absorption), déposée sur un substrat transparent ST d'indice réel $n_2$, par exemple en verre, et immergée dans un milieu ambiant MA d'indice réel $n_0$, par exemple l'air ou l'eau. A titre d'exemple non limitatif on considèrera le cas d'une couche métallique. Sur la couche métallique on dispose un échantillon de référence EO, constitué par une faible épaisseur (nanométrique) d'un matériau transparent (indice réel). L'ensemble constitué par le support SAC (substrat et couche métallique) et l'échantillon est éclairé et observé à travers un objectif LO d'ouverture numérique $n_0 \cdot \sin\theta$ et à travers le substrat. Une goutte d'huile H peut optionnellement être utilisée pour minimiser les réflexions parasites entre l'objectif et le milieu ambiant et entre le milieu ambiant et le substrat. Alternativement, la face avant du substrat peut être recouverte d'un traitement anti-reflet couvrant le spectre ou la partie utile du spectre de l'éclairage.

Comme dans le cas d'un substrat anti-réfléchissant conventionnel (K1=0), le contraste avec lequel l'échantillon est vu dépend fortement de l'indice complexe et de l'épaisseur de la couche, et plus précisément présente un extremum marqué en fonction de l'épaisseur. Cet extremum prend lui-même une valeur extrêmale et change brutalement de signe lorsque les parties réelle et imaginaire de l'indice complexe $\tilde{n}_1 = n_1 - j\kappa_1$ de la couche vérifient la relation :

$$n_1^2 - \kappa_1^2 = n_0 n_2$$

$$(2)$$

La relation (2) peut être considérée une généralisation de la relation (1) au cas d'une couche ayant un indice de réfraction complexe.

Quand $n_0$ et $n_2$ sont fixés, la réflectivité de la surface ne dépend que de l'angle $\theta$, de $\lambda$ (longueur d'onde), de $n_1$ et de $\kappa_1$. Comme elle s'exprime en fonction des coefficients de Fresnel (qui ne font pas intervenir explicitement la longueur d'onde $\lambda$, mais seulement les indices des milieux et les angles) et de l'épaisseur $e_1$ de la couche (qui n'intervient qu'à travers le rapport $\frac{e_1}{\lambda}$), on peut calculer le contraste d'un objet de référence achromatique en fonction de $n_1$, $\kappa_1$ et , ce calcul étant valable pour tous les métaux et toutes les longueurs d'onde. On peut ainsi ajuster le point de fonctionnement en changeant de métal (ou plus généralement, de matériau à indice de réfraction complexe), en changeant d'épaisseur de couche, ou en changeant de longueur d'onde.

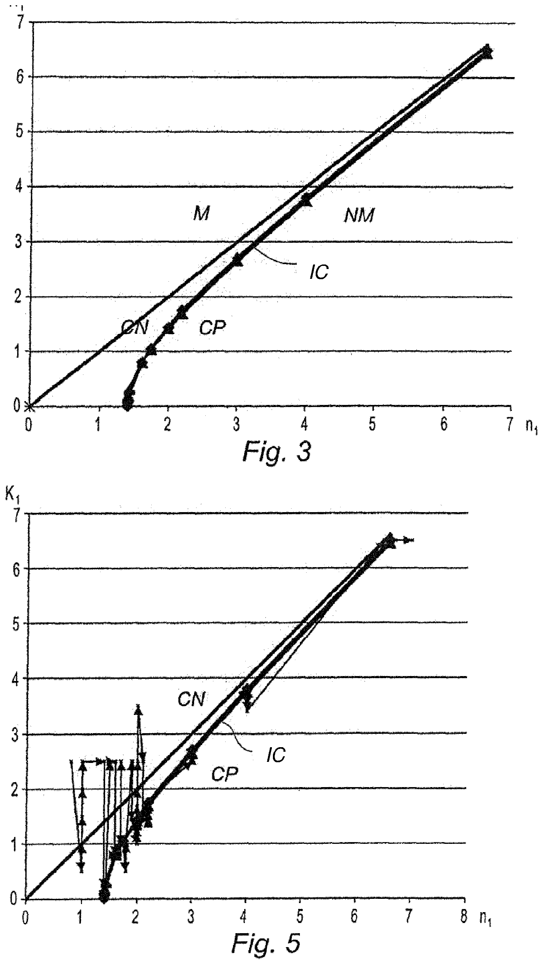

Sur la figure 3, la droite avec pente unitaire correspond à la condition $\kappa_1 = n_1$. La région au-dessus de la droite, marquée par M, correspond aux matériaux métalliques, tandis que la région au-dessous, marquée par NM, correspond aux matériaux non métalliques. La courbe IC est définie par l'équation (2) et correspond à la condition d'inversion de contraste. Au-dessus de cette courbe (région CN) le contraste est négatif, ce qui signifie que l'échantillon apparaît sombre sur fond clair; au-dessous (région CP) le contraste est positif, ce qui signifie que l'échantillon apparaît clair sur fond sombre. La courbe a été calculée pour $n_0 = 1,514$ et $n_2 = 1,33$.

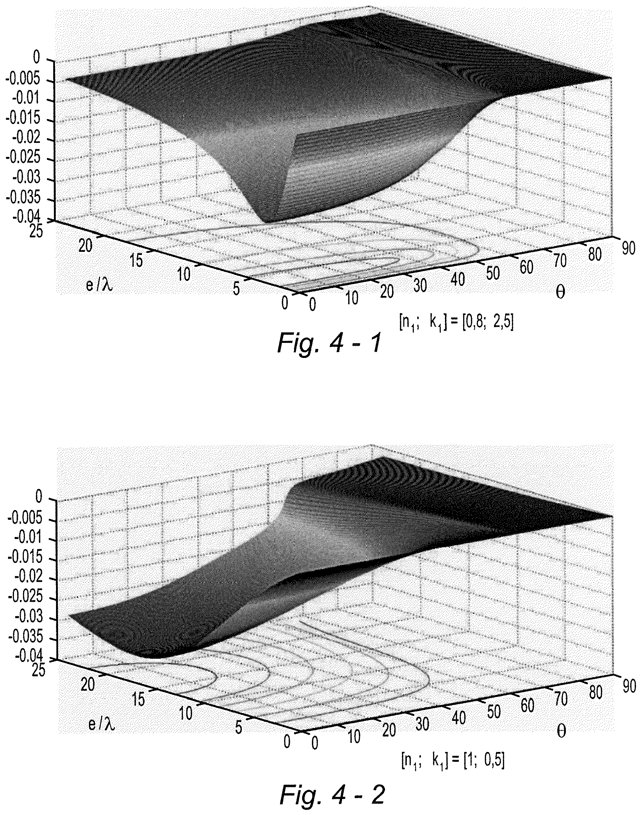

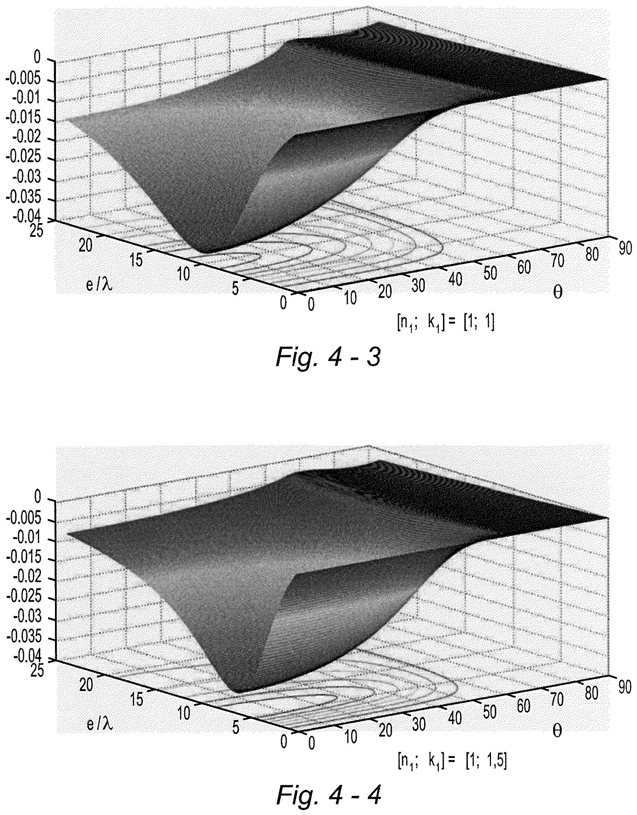

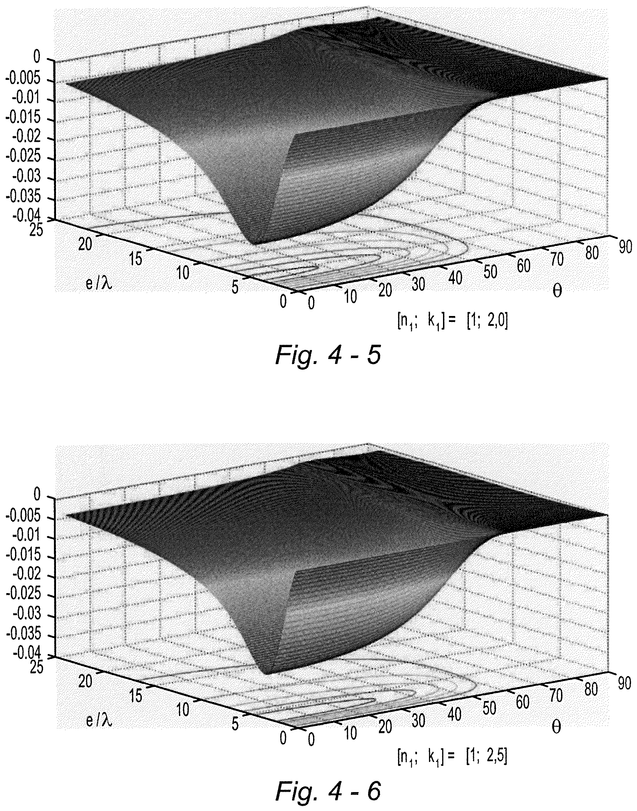

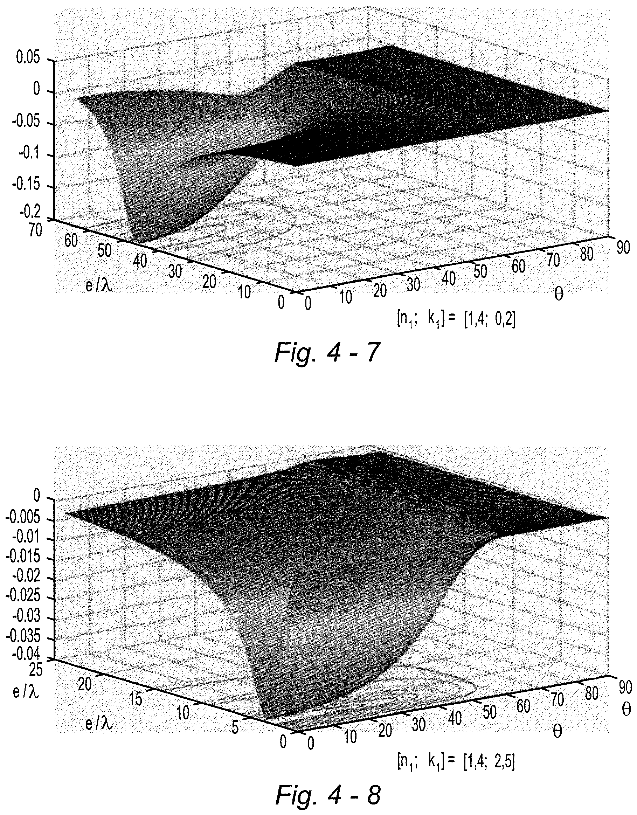

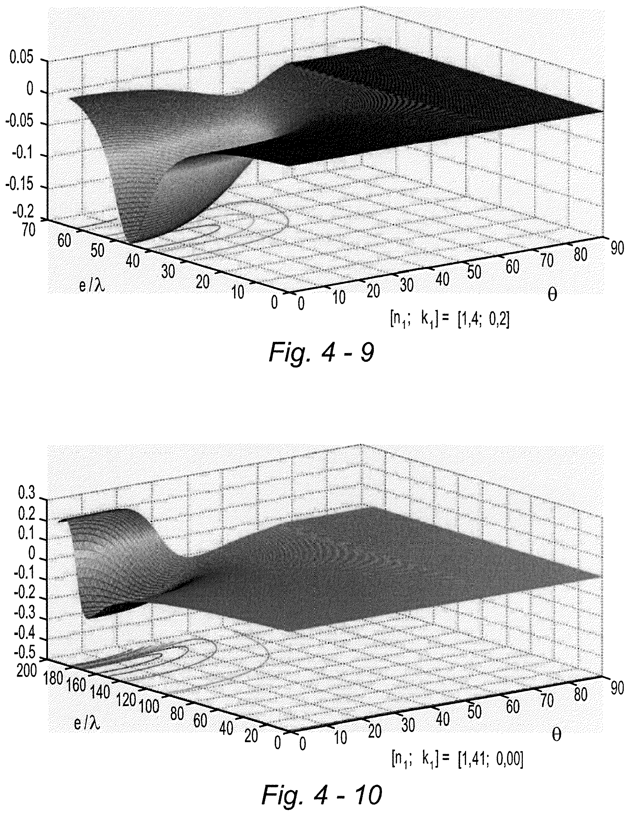

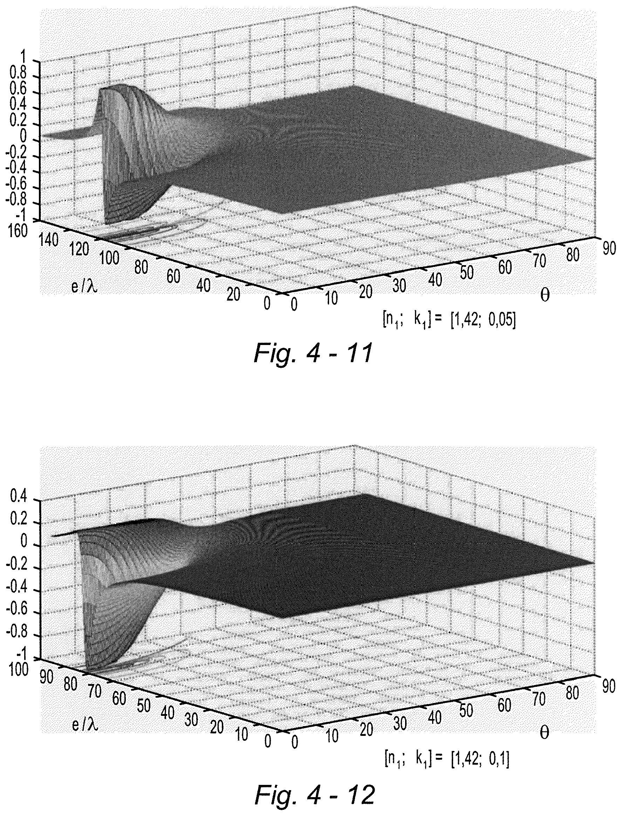

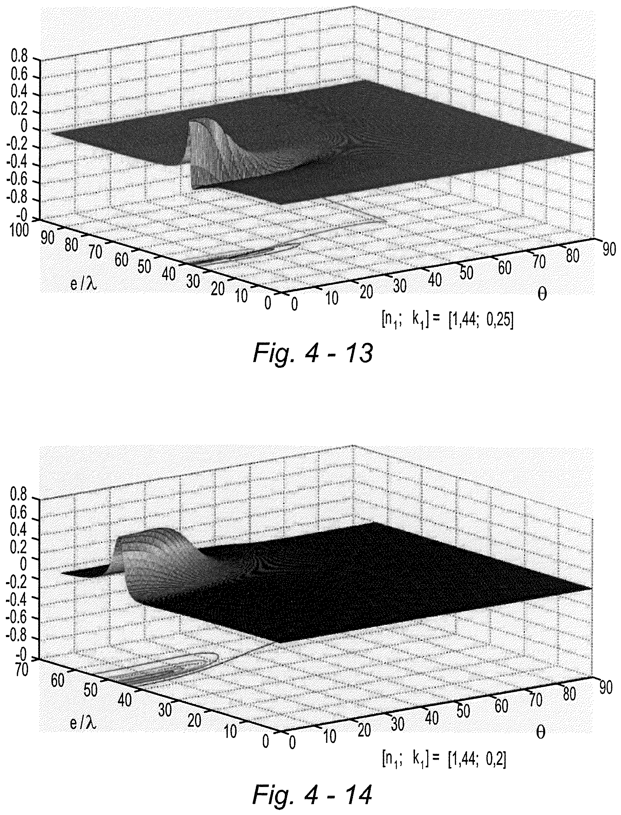

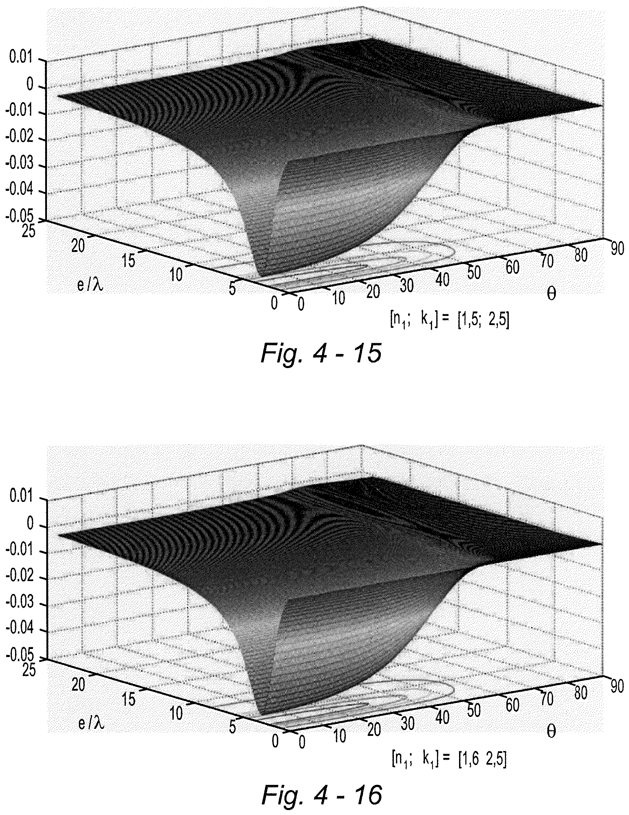

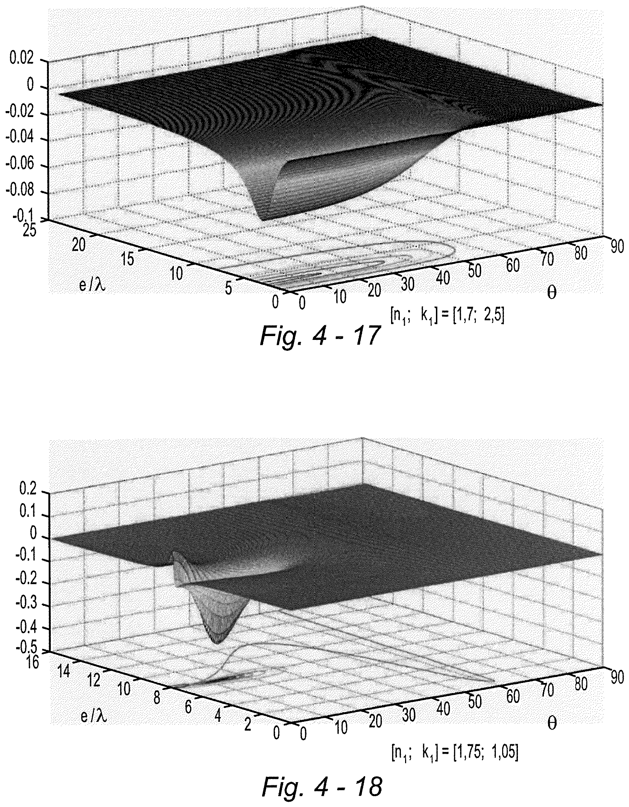

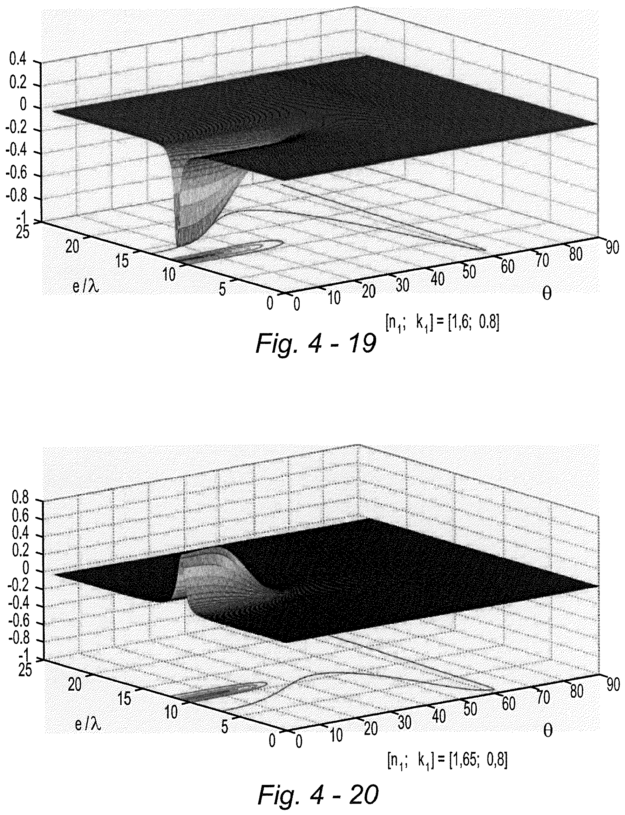

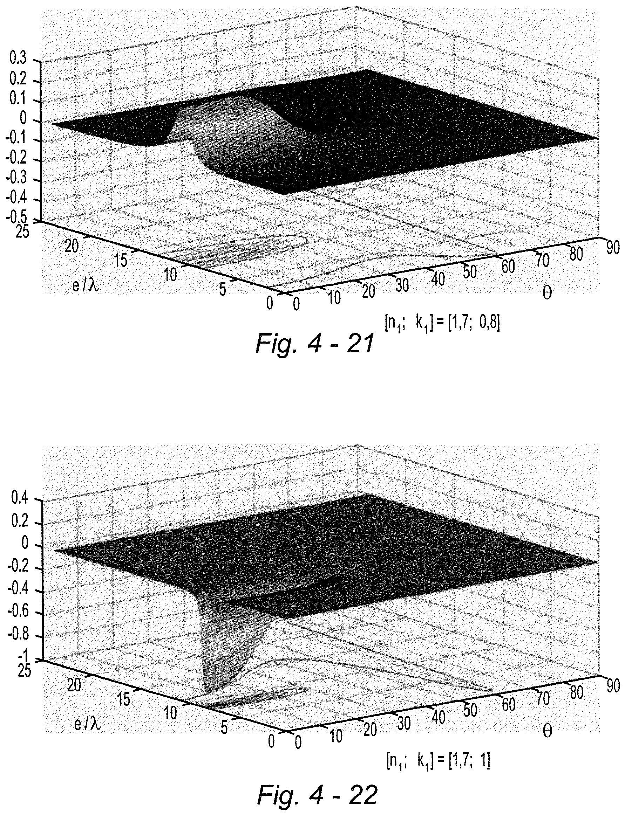

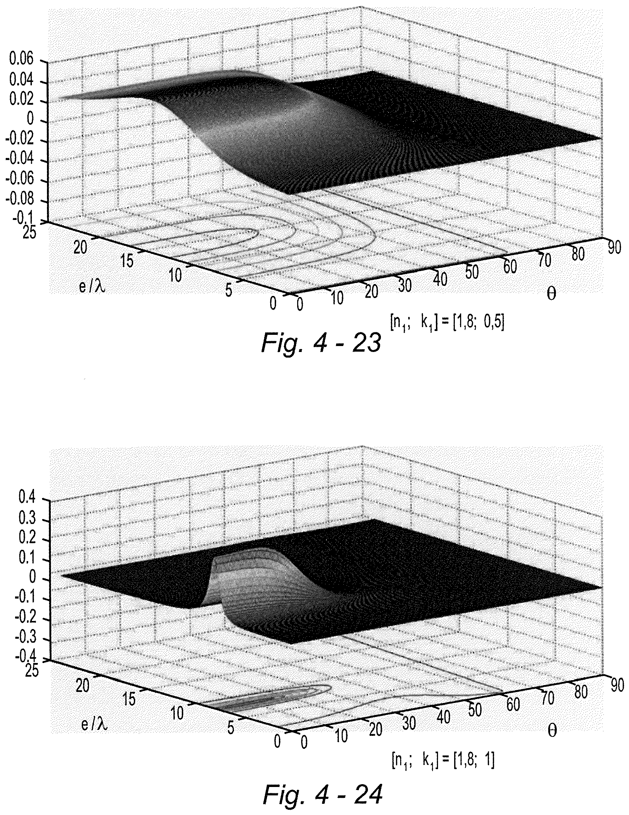

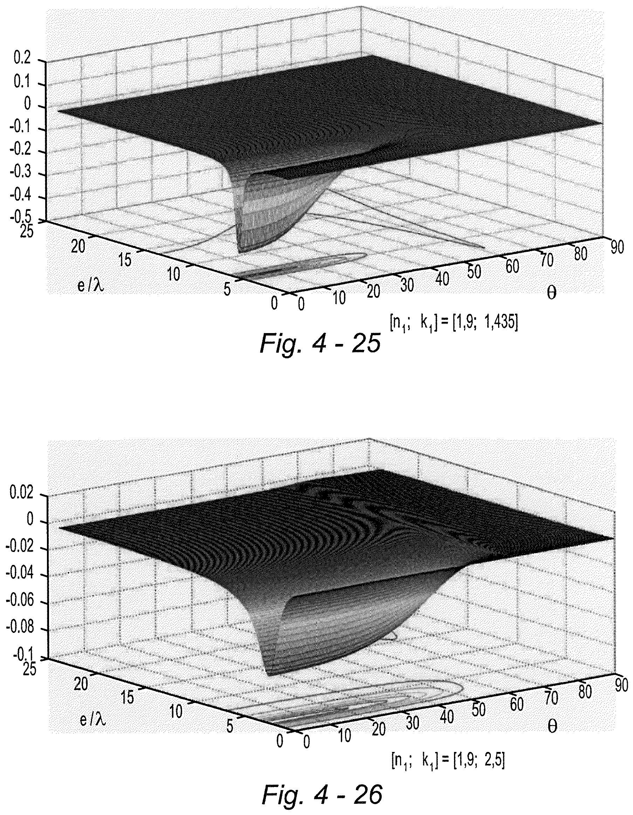

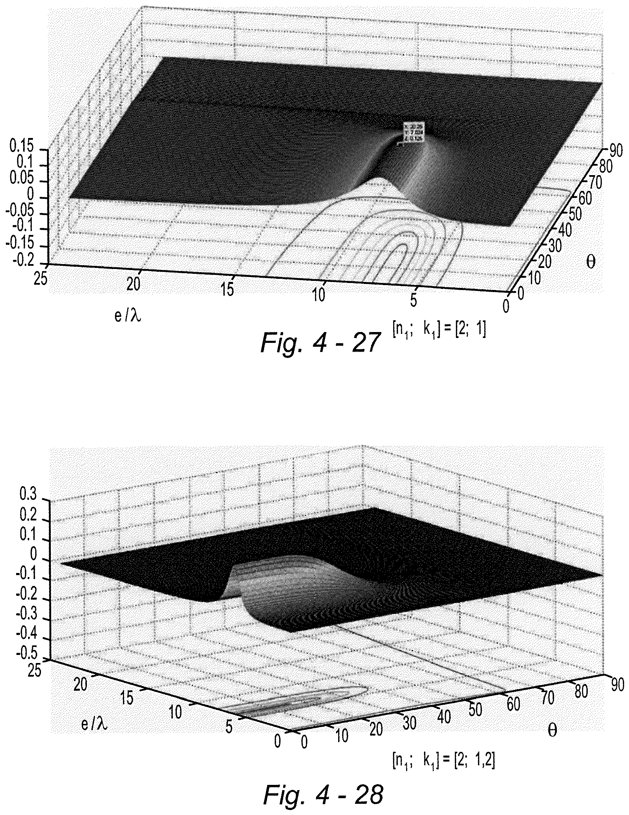

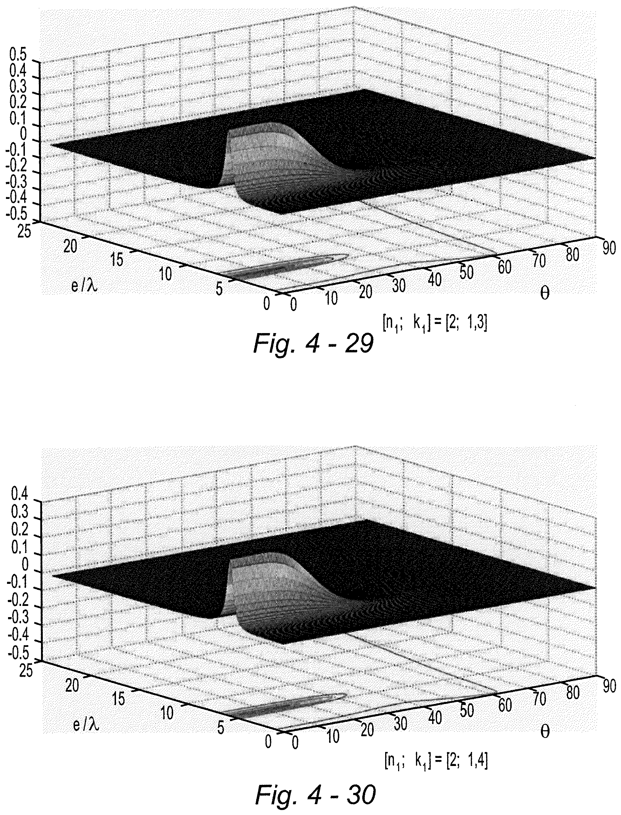

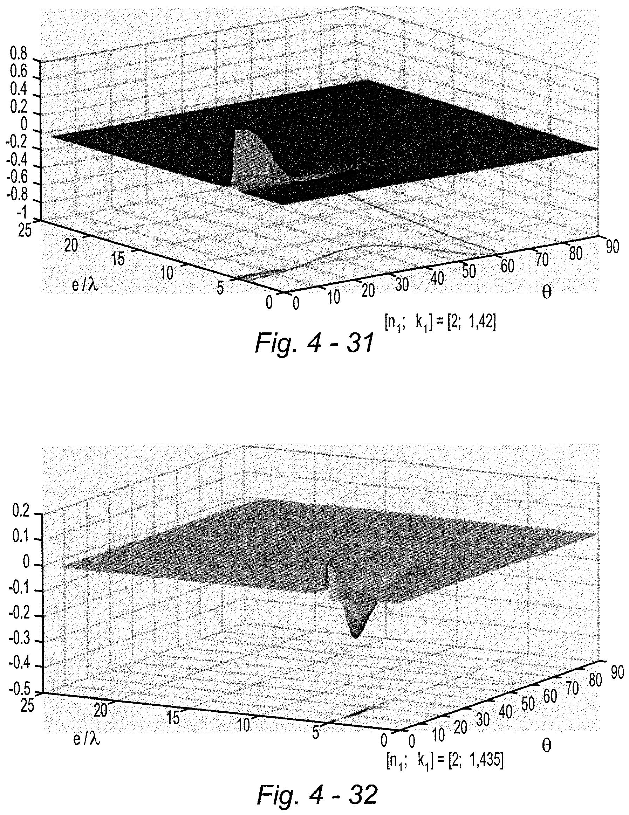

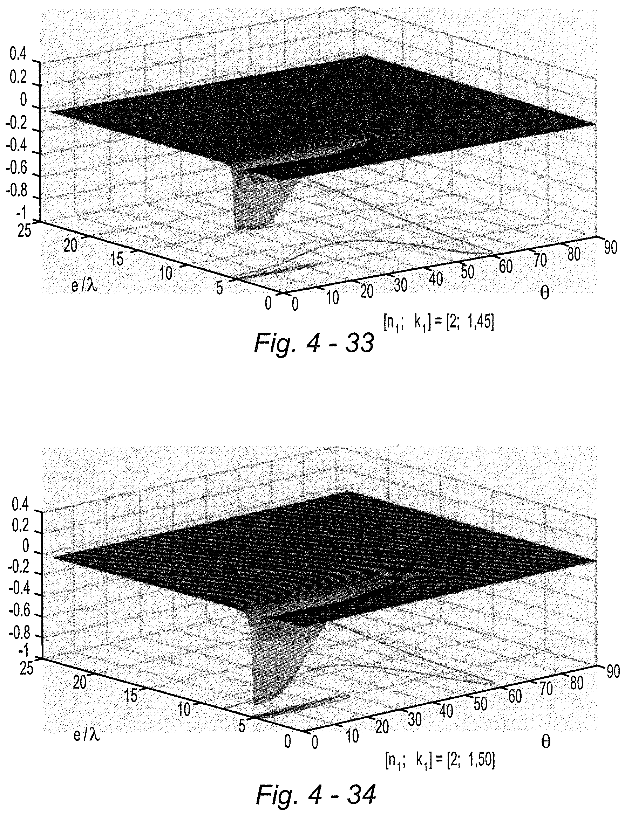

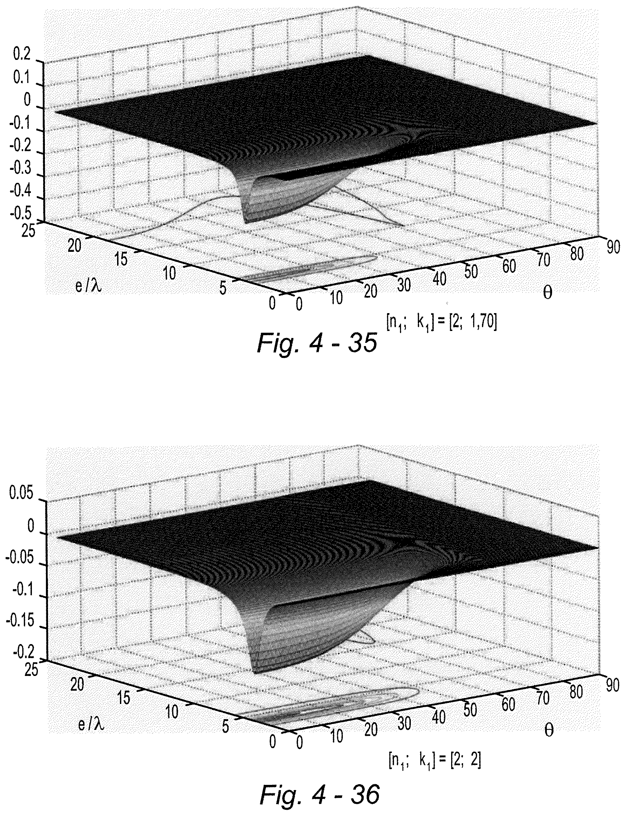

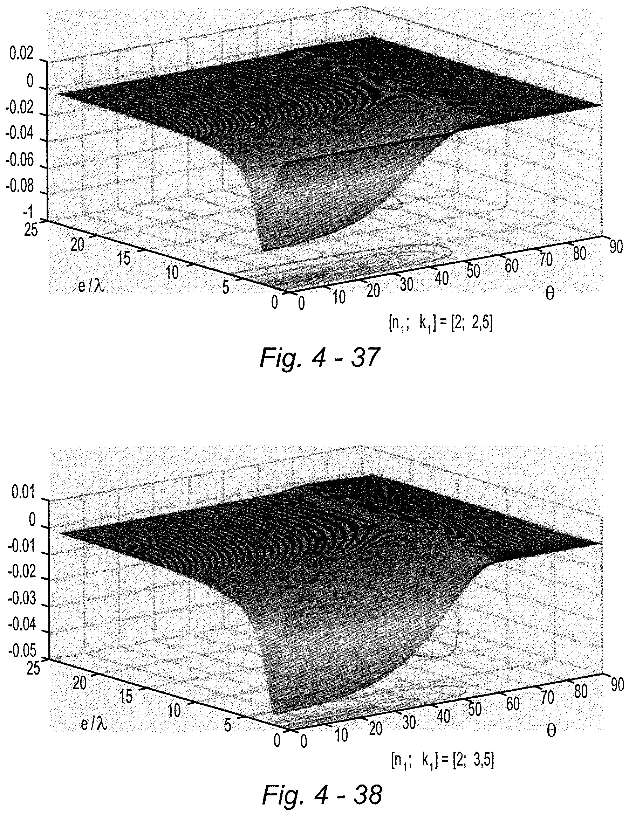

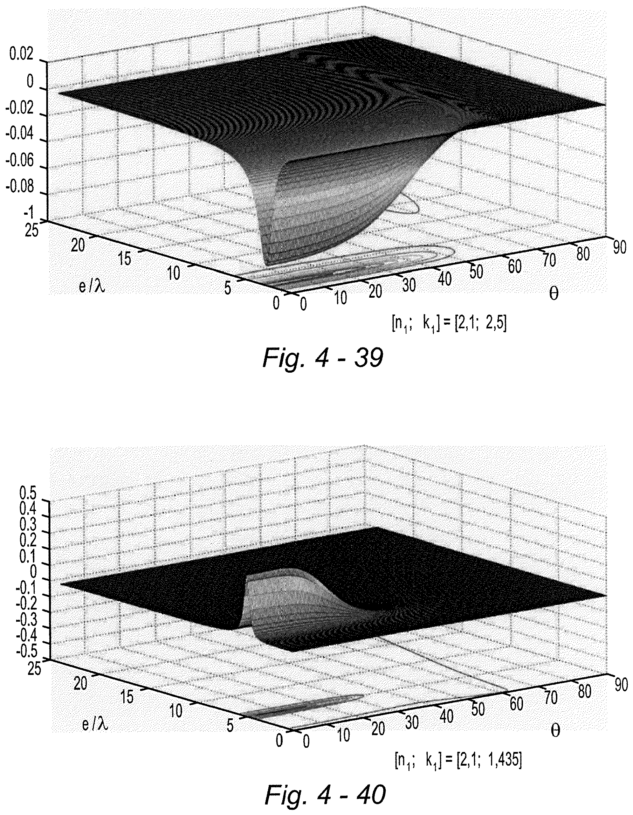

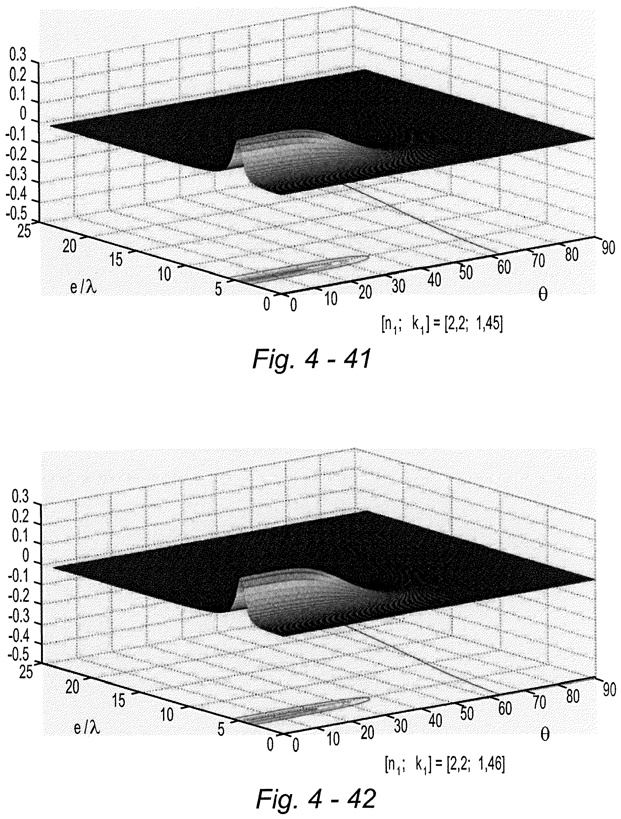

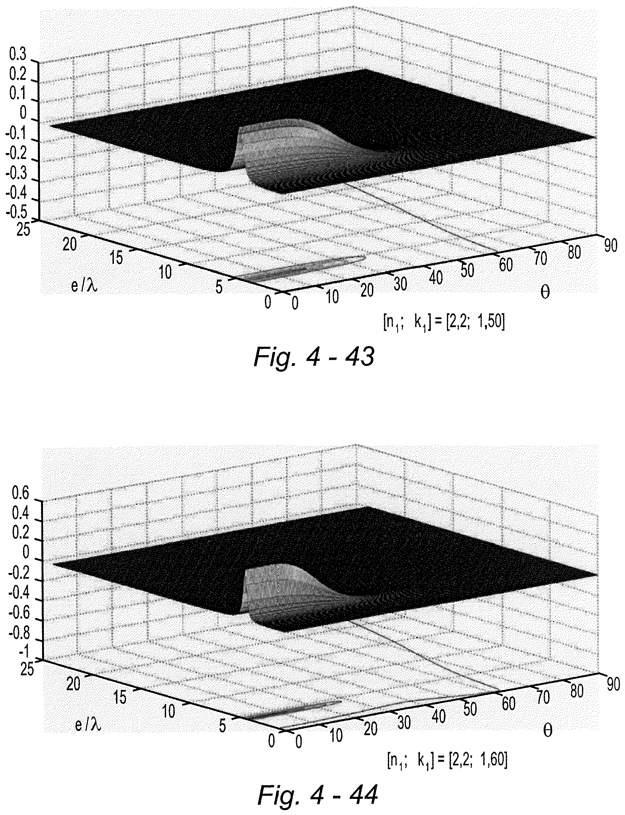

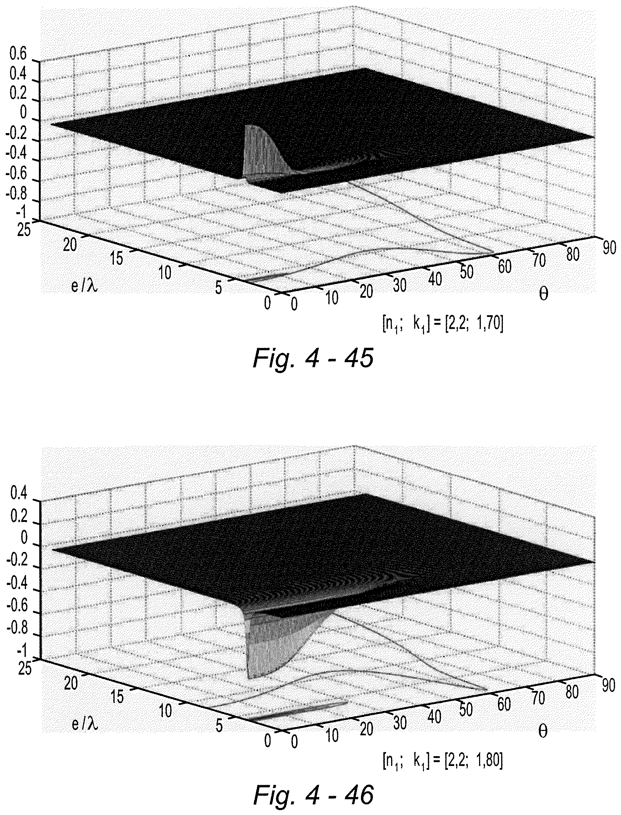

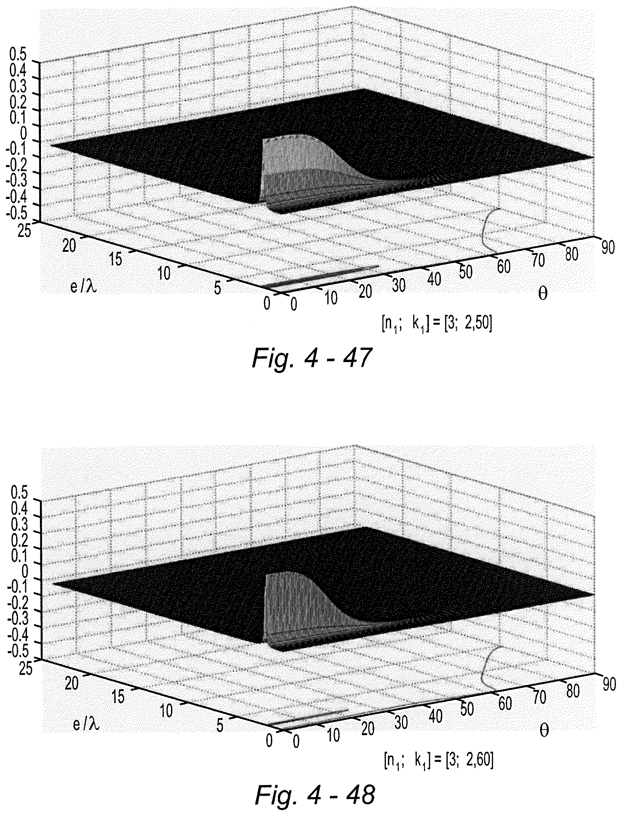

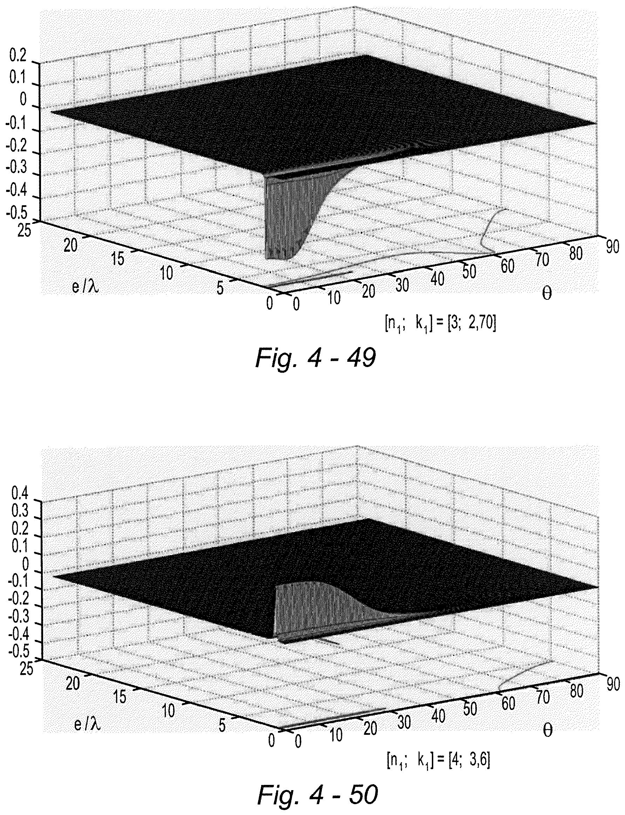

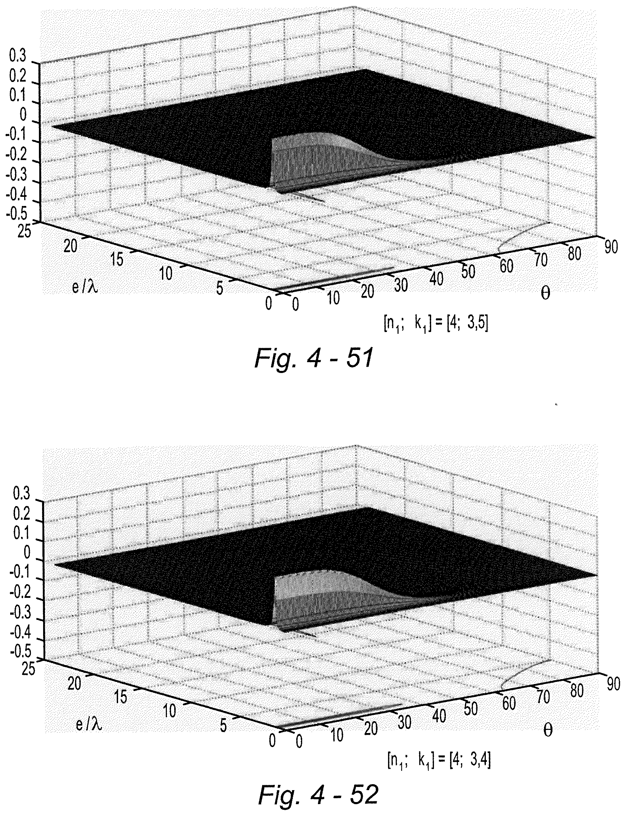

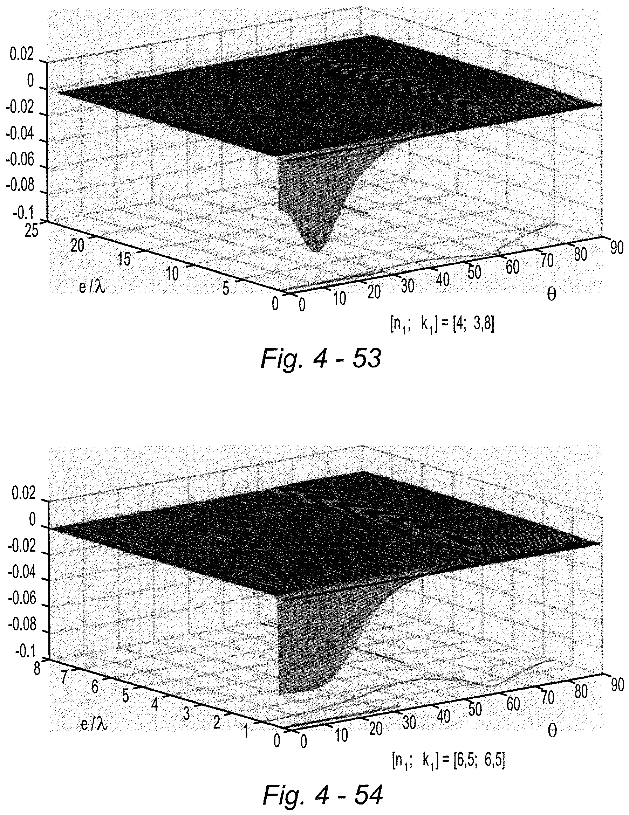

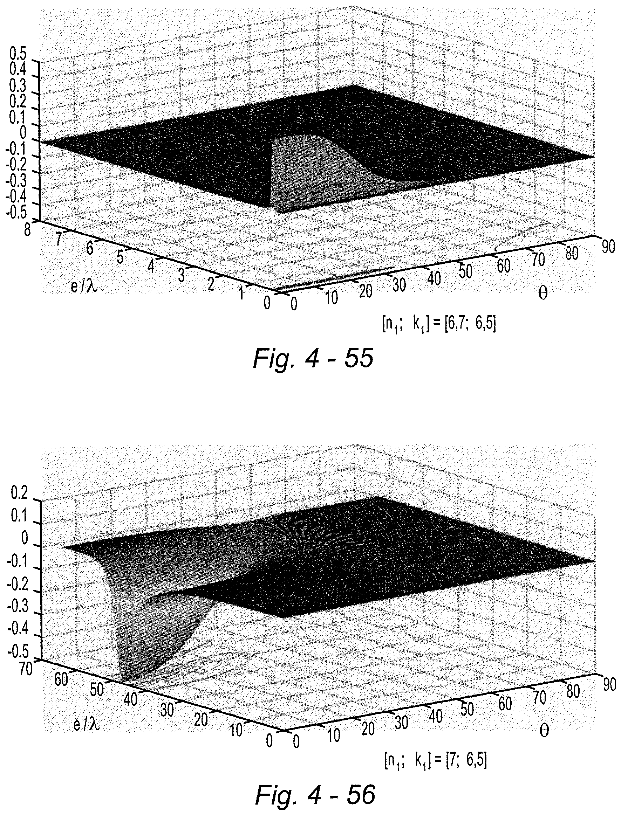

Il est intéressant de tracer les surfaces (figures 4-1 à 4-56) exprimant le contraste avec lequel est observé l'échantillon de référence en fonction de $\theta$ et de $e/\lambda$ pour une pluralité de paires $(n_1, \kappa_1)$ situés à proximité de la courbe IC, de part et d'autre de cette dernière, comme illustré sur la figure 5. On peut observer sur les figures 4-1 à 4-56 que la plupart de ces surfaces présentent soit des vallées (en cas de contraste négatif), soit des «dorsales » (en cas de contraste positif), sensiblement alignées selon l'axe représentatif de l'angle $\theta$. Cela signifie que, pour une valeur définie de l'épaisseur normalisée *e/λ* (correspondant à la crête de la dorsale ou au fond de la vallée, c'est-à-dire au thalweg), dite épaisseur critique $(e/\lambda)_c$, le contraste présente une valeur maximale qui ne dépend pas - ou faiblement - de $\theta$ tant que cet angle se maintient dans une plage définie (s'étendant, le plus souvent, de 0° à 40 - 50°). Cela signifie que, pour ladite valeur critique de l'épaisseur normalisée $(e/\lambda)_c$, l'éclairage et l'observation à travers un objectif n'entraînent pas une perte de contraste prononcée comme par exemple dans la technique SEEC. La valeur $e/\lambda$ maximise (en valeur absolue) le contraste intégré sur tous les angles d'incidence contenus dans le cône d'éclairage. Les valeurs de contraste npeuvent varier de plus de 10% à quelques pour mille, ce qui est encore exploitable. Dans le cas des figures 4-1 à 4-56 on a choisi $n_0$=1,514 et $n_2$=1,33, ce qui correspond à un substrat en verre et à une observation en immersion dans l'eau. D'une manière générale, l'épaisseur critique augmente avec l'indice optique du substrat.

De manière empirique, on peut écrire la relation qui lie l'épaisseur critique $(e/\lambda)_c$ à la partie imaginaire de l'indice de la couche amplificatrice de contraste sous la forme :

$$(e/\lambda)_c = C/\kappa \tag{3}$$

où C est une constante qui, dans l'exemple considéré ici vaut 0,01. Il reste encore un paramètre de dimensionnement libre, $n_1$, qui peut être utilisé pour choisir le meilleur compromis entre ouverture numérique admissible (longueur de la crête ou du thalweg) et contraste (hauteur de la crête ou profondeur de la vallée) pour une application donnée. En effet, plus la paire ($n_1$ ; $\kappa_1$) est proche de la ligne d'inversion de contraste, plus le contraste sera élevé à faible ouverture (ou pour un angle d'incidence déterminé), mais plus il décroîtra rapidement avec l'ouverture numérique de l'objectif.

Une crête ou thalweg est également observée en cas d'observation en lumière polarisée (entre un polariseur et un analyseur croisés, ou en tout cas formant un angle entre eux), et notamment pour $\kappa_1 > n_1$, mais l'épaisseur critique et la condition reliant les indices de réfraction entre eux sont différentes que dans le cas d'observation en lumière non polarisée.

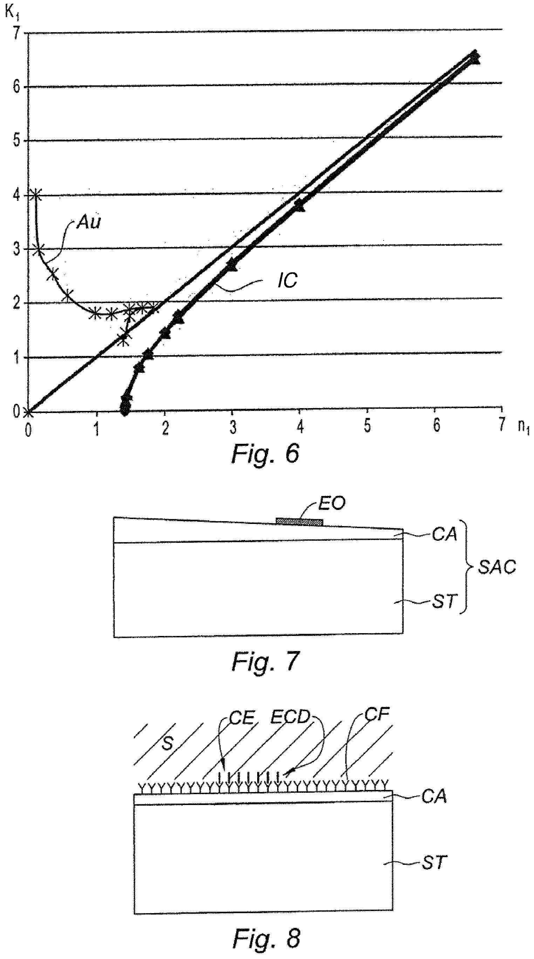

Comme montré sur la figure 7, il est possible d'utiliser un support ayant une couche amplificatrice de contraste présentant un gradient d'épaisseur. La condition (3) sera satisfaite, pour au moins une longueur d'onde d'éclairage, en une région déterminée du support, qui pourra être trouvée par simple observation.

Pour se déplacer dans le plan ($n_1$ ; $\kappa_1$) on peut jouer sur la composition de la couche amplificatrice de contraste - qui peut même être en fait une multicouche, ce qui donne une grande liberté au concepteur-et/ou sur la longueur d'onde d'éclairage dans toute la gamme autorisée par les éléments optiques du système, généralement du proche UV ($\lambda$= 200 nm) au proche infrarouge ($\lambda$= 2 $\mu$m) Les longueurs d'onde UV et Infrarouge présentent l'avantage de permettre l'exploitation des bandes d'absorption naturelles des molécules visualisées pour obtenir un meilleur contraste, et le cas échéant un contraste spécifique de l'espèce recherchée). A titre d'exemple, la courbe Au sur la figure 6 montre les valeurs de $n_1$ et $\kappa_1$ pour une couche d'or en fonction de la longueur d'onde d'éclairage qui varie dans la plage 350-750 nm.

Le cas de l'or est particulièrement intéressant car il constitue une norme pour les capteurs biologiques. Le cas d'un film d'or constitué de nanoparticules est particulièrement avantageux car on peut régler $n_1$ et K1 en jouant sur la concentration et la densité des particules qui le constituent.

Le principe que l'on vient de décrire permet l'observation d'objets microscopiques et de faible contraste. Il permet également la réalisation de biopuces pour la détection et/ou le dosage d'espèces chimiques ou biologique. Par exemple, comme illustré sur la figure 8, il est possible de déposer une couche de fonctionnalisation CF sur la couche amplificatrice de contraste. La couche de fonctionnalisation est mise en contact avec une solution S, par exemple aqueuse, contenant l'espèce chimique ou biologique à détecter ECO. Cette dernière est fixée par la couche de fonctionnalisation et forme une couche mince supplémentaire CE, constituant l'échantillon à observer. En pratique, dans le cas d'une biopuce, on déposera plusieurs plots de fonctionnalisation différents, permettant de fixer sélectivement des espèces chimiques ou biologiques différentes. En observant la biopuce au microscope, dans les conditions décrites ci-dessus, on peut identifier facilement les espèces effectivement présentes dans la solution.

La sensibilité de la détection sera considérablement améliorée si les espèces fixées sont elles-mêmes au moins légèrement optiquement absorbantes pour la longueur d'onde utile de l'éclairage, par exemple si la partie imaginaire de leur indice est supérieure à 0,0001, et de préférence supérieure à 0,001, et de préférence supérieure à 0,01.

Le contraste est également augmenté si la couche amplificatrice de contraste n'est déposée qu'en correspondance desdits plots.

De préférence, en dehors des plots on peut prévoir une couche de passivation empêchant la fixation de toute espèce chimique ou biologique contenue dans ladite solution. On peut utiliser par exemple un polyéthylène glycol, un polymère fluoré, ou un alkyl fluoré, par exemple fonctionnalisés par des thiols dans le cas de l'or. Cette couche de passivation peut être déposée en phase vapeur après la fabrication des plots.

Lorsqu'on souhaite détecter ou déposer des espèces chimiques ou biologiques, il est également possible d'utiliser un substrat uniquement pourvu de couche de fonctionnalisation.

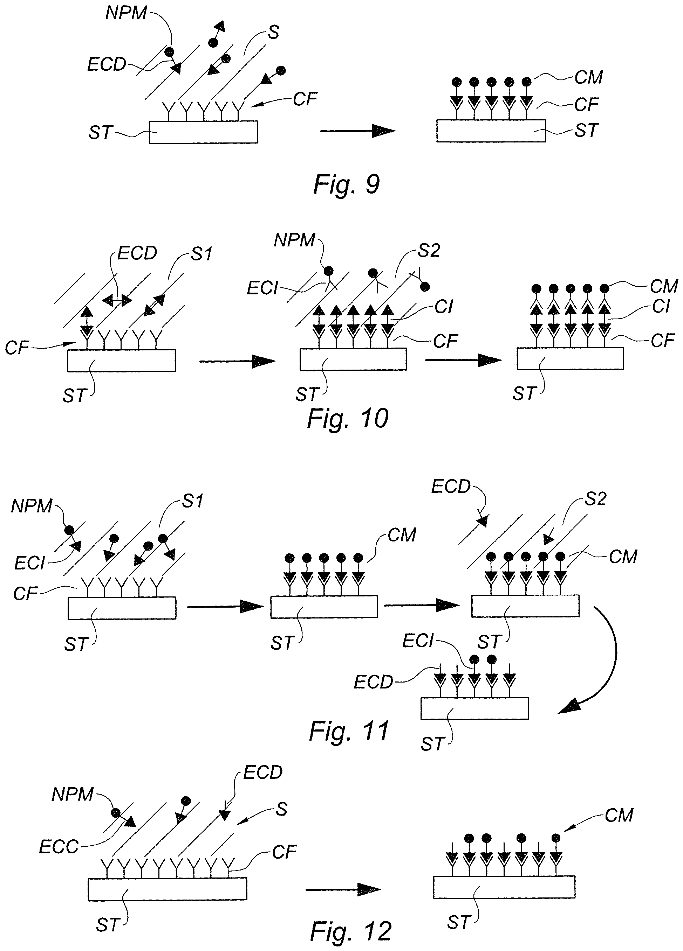

Selon un premier mode de réalisation, illustré sur la figure 9, **la** couche de fonctionnalisation est mise en contact avec une solution contenant une espèce chimique ou biologique ECO à détecter ou doser, marquée par des nanoparticules métalliques NPM et susceptible de se fixer sur ladite couche de fonctionnalisation de manière à former une couche métallique CM. Cette couche peut être en réalité discontinue, mais elle apparaît continue à l'échelle de la longueur d'onde de la lumière visible (plusieurs centaines de nanomètres), avec une épaisseur effective qui peut être une fraction du diamètre de la nanoparticule, et avec un indice de réfraction effectif. L'observation se fait de la manière décrite plus haut, la couche métallique ainsi constituée servant à la fois de couche amplificatrice de contraste et d'échantillon. Pour un temps de contact déterminé entre la solution et la couche fonctionnalisée, l'épaisseur de la couche métallique dépend de la teneur en espèce chimique ou biologique, ce qui permet de réaliser un dosage.

En variante, les nanoparticules métalliques peuvent être remplacées par un marqueur absorbant, par exemple une molécule fluorescente (à noter que la fluorescence, en soi, n'est pas exploitée, mais une molécule fluorescente est fortement absorbante).

L'inconvénient du premier mode de réalisation est de ne permettre que la détection d'une espèce chimique ou biologique marquée. Les modes de réalisation suivants ne présentent pas cet inconvénient.

Selon le deuxième mode de réalisation (figure 10), la couche fonctionnalisée est placée en contact avec une première solution S1 contenant l'espèce chimique ou biologique à détecter ou doser, de manière à former une couche dite intermédiaire CI. Cette couche intermédiaire n'est pas observable. Pour la révéler, on la met en contact avec une deuxième solution S2, contenant une espèce chimique ou biologique dite auxiliaire ECA, marquée par des nanoparticules métalliques (ou un marqueur absorbant) et susceptible de se fixer sur ladite couche intermédiaire pour former la couche métallique (ou absorbante) CM.

La technique peut être quantitative si l'espèce à détecter est présente en quantité insuffisante pour saturer la couche de fonctionnalisation et, par contre, l'espèce auxiliaire est présente en excès. Dans ce cas, en effet, l'épaisseur et l'indice effectifs de la couche CM - et donc l'intensité du signal lumineux observé -dépendrons de la concentration de l'espèce à détecter.

Ce deuxième mode de réalisation ne peut être utilisé que si l'espèce chimique ou biologique à détecter présente au moins deux sites actifs, il ne s'applique donc pas, par exemple, aux haptènes. En outre, il est assez complexe à mettre en œuvre.

Les modes de réalisation suivants ne présentent pas cet inconvénient.

Selon le troisième mode de réalisation (figure 11), la couche fonctionnalisée est placée en contact avec une première solution (S1) contenant une espèce chimique ou biologique, dite espèce intermédiaire ECI, marquée par des nanoparticules métalliques ou un marqueur absorbant et susceptible de se fixer sur ladite couche de fonctionnalisation pour former ladite couche métallique ou absorbante (CM) continue ou discontinue. Ensuite, l'ensemble ainsi obtenu est mis en contact avec une deuxième solution (S2) contenant l'espèce chimique ou biologique à détecter ou doser, laquelle présente une affinité avec ladite couche de fonctionnalisation supérieure à celle de ladite espèce intermédiaire. Ainsi, l'espèce intermédiaire est déplacée et ladite couche métallique ou absorbante est supprimée au moins en partie, ce qui se traduit par une diminution du signal lumineux. La technique est plutôt qualitative que quantitative, elle convient donc mieux à la détection qu'au dosage. Un avantage de cette approche est que ses deux étapes peuvent être dissociées : les supports peuvent être fournis avec la couche CM déjà formée, prêts à être utilisés comme capteurs chimiques ou biologiques.

Selon un quatrième mode de réalisation (figure 12), l'on place ladite couche de fonctionnalisation en contact avec une solution S contenant l'espèce chimique ou biologique à doser, ainsi que ladite espèce chimique ou biologique concurrente ECC, l'une des deux espèces (de préférence l'espèce concurrente) étant marquée par des nanoparticules métalliques ou un marqueur absorbant. Ainsi, l'on obtient une couche métallique ou absorbante CM dont l'épaisseur effective et l'indice effectif dépendent du rapport entre la concentration de ladite espèce chimique ou biologique concurrente et celle de la dite espèce chimique ou biologique à doser. Comme dans les autres modes de réalisation, le signal dépend de cette épaisseur effective et de cet indice effectif.

Les espèces chimiques ou biologiques peuvent être, par exemple, anticorps, antigènes, protéines, microorganismes, etc.

Au lieu d'être métallique ou absorbant, le marqueur peut être diffusant. En effet, il est connu que l'effet de la diffusion peut être exprimé par un indice de réfraction ayant une partie imaginaire.

Les techniques de détection ou dosage décrites ci-dessus s'appliquent également lorsque la couche de fonctionnalisation est déposée sur une couche d'amplification de contraste telle que décrite plus haut. La couche fonctionnalisée, et le cas échéant la couche d'amplification de contraste peuvent être structurés en plots, et la surface en dehors de ces plots peut être passivée, comme expliqué plus haut.

## Revendications

**1.** Procédé d'observation d'un échantillon (EO) comportant les étapes consistant à :

    a) Procurer un support d'échantillon (SAC) comprenant un substrat transparent (ST) sur lequel est déposée au moins une couche (CA), dite amplificatrice de contraste, présentant un indice de réfraction complexe avec une partie imaginaire $\kappa$ supérieure ou égale à 0,001 et préférentiellement à 0,01 ;
    b) Disposer l'échantillon à observer sur ladite couche amplificatrice de contraste ;
    c) Diriger sur ledit échantillon, à travers ledit substrat, un faisceau (F) de lumière spatialement incohérente, focalisé de manière à former un cône d'éclairage présentant un demi-angle d'ouverture 8 supérieure ou égale à 20°; et

d) Observer ledit échantillon à travers un objectif (LO) et ledit substrat; **caractérisé en ce que** :

ladite couche amplificatrice de contraste étant dimensionnée de telle manière que son épaisseur, ou son épaisseur locale dans une région déterminée, optimise, pour au moins une longueur d'onde d'éclairage, le contraste intégré sur ledit cône d'éclairage avec lequel serait observé un échantillon de référence constitué par une épaisseur nanométrique d'un matériau transparent,

et **en ce que** :

ledit faisceau de lumière est non polarisé.

2. Procédé d'observation d'un échantillon selon la revendication 1, dans lequel ladite couche amplificatrice de contraste est métallique.

3. Procédé d'observation d'un échantillon selon l'une des revendications précédentes, dans lequel le demi-angle d'ouverture dudit cône d'éclairage est compris entre 20° et 75°, de préférence entre 30° et 70° et de manière encore préférée entre 40° et 65°, l'axe dudit cône étant perpendiculaire audit substrat.

4. Procédé d'observation d'un échantillon selon l'une des revendications précédentes, dans lequel ledit objectif est utilisé à la fois pour éclairer et pour observer ledit échantillon.

5. Procédé d'observation d'un échantillon selon l'une des revendications précédentes, dans lequel la couche amplificatrice de contraste et ledit échantillon sont immergés dans l'eau ou dans une solution aqueuse pendant l'étape d).

6. Procédé d'observation d'un échantillon selon l'une des revendications précédentes, dans lequel ladite couche amplificatrice de contraste présente un gradient d'épaisseur.

7. Procédé d'observation d'un échantillon selon l'une des revendications précédentes, comportant une étape préliminaire de dimensionnement de ladite couche amplificatrice de contraste comprenant :

- le calcul d'une surface représentant le contraste avec lequel serait observé ledit échantillon de référence en fonction de l'épaisseur de ladite couche, normalisée par rapport à une longueur d'onde d'éclairage, et du demi-angle d'ouverture $\theta$ du faisceau d'éclairage ;
- l'identification d'une crête ou thalweg de ladite surface, présentant une orientation approximativement parallèle à l'axe représentant ledit demi-angle d'ouverture $\theta$ ;
- l'identification d'une valeur, ou plage de valeurs, d'épaisseur de ladite couche correspondant à ladite crête ou thalweg ;

l'épaisseur ou une épaisseur locale de ladite couche amplificatrice de contraste étant choisie égale à la valeur, ou à l'intérieur de la plage de valeurs, ainsi identifiée.

8. Procédé d'observation d'un échantillon selon l'une des revendications précédentes, dans lequel ledit support d'échantillon présente, au-dessus de ladite couche d'amplification de contraste, une couche de fonctionnalisation (CF) susceptible de fixer au moins une espèce chimique ou biologique (ECO), le procédé comportant également une étape de mise en contact de la dite couche de fonctionnalisation avec une solution de ladite espèce chimique ou biologique à fixer, moyennant quoi ladite espèce chimique ou biologique forme une couche (CE) au-dessus de ladite couche de fonctionnalisation, constituant l'échantillon à observer, de préférence dans lequel ladite ou au moins une espèce chimique ou biologique est absorbante, présentant un indice de réfraction complexe avec une partie imaginaire $\kappa$ supérieure ou égale à 0,0001, ou diffusante, et de préférence dans lequel ladite couche de fonctionnalisation se présente sous la forme d'une pluralité de plots susceptibles de fixer des espèces chimiques ou biologiques différentes, ladite couche de contraste n'étant optionnellement déposée qu'en correspondance desdits plots et ledit support comprenant optionnellement, en dehors desdits plots, une couche de passivation empêchant la fixation de toute espèce chimique ou biologique contenue dans ladite solution.

9. Procédé de détection ou dosage d'au moins une espèce chimique ou biologique comportant les étapes consistant à :

a) Procurer un substrat transparent (ST) sur lequel est déposée au moins une couche de fonctionnalisation (CF) susceptible de fixer au moins une espèce chimique ou biologique ;

b) Placer ladite couche de fonctionnalisation en contact avec au moins une solution contenant une espèce chimique ou biologique marquée par des nanoparticules métalliques ou un marqueur absorbant ou diffusant, ladite espèce étant susceptible de se fixer sur ladite couche de fonctionnalisation, soit directement, soit par l'intermédiaire d'une ou plusieurs autres espèces chimiques ou biologiques, moyennant quoi lesdites particules forment une couche métallique, absorbante ou diffusante (CM) continue ou discontinue ;

c) Diriger sur l'ensemble constitué par au moins les dites couches de fonctionnalisation et métallique, absorbante ou diffusante, à travers ledit substrat, un faisceau (F) de lumière spatialement incohérente, focalisé de manière à former un cône d'éclairage présentant un demi-angle d'ouverture θ supérieure ou égale à 20° ; et

d) Observer ledit ensemble à travers un objectif (LO) et travers ledit substrat,

**caractérisé en ce que** ledit faisceau de lumière est non polarisé.

10. Procédé de détection ou dosage d'au moins une espèce chimique ou biologique selon la revendication 9, dans lequel, lors de l'étape b), on place ladite couche de fonctionnalisation en contact avec une solution (S) contenant l'espèce chimique ou biologique à détecter ou doser (ECO), marquée par des nanoparticules métalliques (NPM) ou un marqueur absorbant ou diffusant, ladite espèce étant susceptible de se fixer sur ladite couche de fonctionnalisation pour former ladite couche métallique (CM) continue ou discontinue.

11. Procédé de détection ou dosage d'au moins une espèce chimique ou biologique selon la revendication 9, dans lequel ladite étape b) comprend les sous-étapes consistant à :

b1) Placer ladite couche de fonctionnalisation en contact avec une première solution (S1) contenant l'espèce chimique ou biologique à détecter ou doser, de manière à former une couche dite intermédiaire (CI) ; et

b2) Placer ladite couche intermédiaire en contact avec une deuxième solution (S2), contenant une espèce chimique ou biologique dite auxiliaire (ECA), marquée par des nanoparticules métalliques ou un marqueur absorbant ou diffusant et susceptible de se fixer sur ladite couche intermédiaire pour former ladite couche métallique, absorbante ou diffusante (CM) continue ou discontinue.

12. Procédé de détection ou dosage d'au moins une espèce chimique ou biologique selon la revendication 9, dans lequel ladite étape b) comprend les sous-étapes consistant à :

b1) Placer ladite couche de fonctionnalisation en contact avec une première solution (S1) contenant une espèce chimique ou biologique, dite espèce intermédiaire (ECI), marquée par des nanoparticules métalliques ou un marqueur absorbant ou diffusant et susceptible de se fixer sur ladite couche de fonctionnalisation pour former ladite couche métallique, absorbante ou diffusante (CM) continue ou discontinue ; et

b2) Placer ladite couche de fonctionnalisation et ladite couche métallique, absorbante ou diffusante en contact avec une deuxième solution (S2) contenant ladite espèce chimique ou biologique à détecter ou doser, laquelle présente une affinité avec ladite couche de fonctionnalisation supérieure à celle de ladite espèce intermédiaire, moyennant quoi ladite espèce intermédiaire est déplacée et ladite couche métallique, absorbante ou diffusante est supprimée au moins en partie.

13. Procédé de détection ou dosage d'au moins une espèce chimique ou biologique selon la revendication 9, dans lequel, lors de ladite étape b), on place ladite couche de fonctionnalisation en contact avec une solution (S) contenant l'espèce chimique ou biologique à doser, ainsi que ladite espèce chimique ou biologique concurrente (ECC), l'une des deux espèces étant marquée par des nanoparticules métalliques ou un marqueur absorbant ou diffusant, moyennant quoi on obtient une couche métallique, absorbante ou diffusante (CM) continue ou discontinue dont l'épaisseur effective dépend du rapport entre la concentration de ladite espèce chimique ou biologique concurrente et celle de la dite espèce chimique ou biologique à doser.

14. Procédé de détection ou dosage selon l'une des revendications 9 à 13, dans lequel le demi-angle d'ouverture dudit cône d'éclairage est compris entre 20° et 75°, de préférence entre 30° et 70° et de manière encore préférée entre 40° et 65°, l'axe dudit cône étant perpendiculaire audit substrat, de préférence dans lequel un même objectif est utilisé pour éclairer et pour observer ledit échantillon, de préférence dans lequel ladite couche de fonctionnalisation se présente sous la forme d'une pluralité de plots disposés sur la surface dudit substrat et susceptibles de fixer des espèces chimiques ou biologiques différentes, ledit support comprenant optionnellement, en dehors desdits plots, une couche de passivation empêchant la fixation de toute espèce chimique ou biologique contenue dans ladite solution, et de préférence dans lequel une couche dite amplificatrice de contraste, présentant un indice de réfraction complexe avec une partie imaginaire κ supérieure ou égale à 0,001 et préférentiellement à 0,01, est interposée entre ledit substrat et ladite couche de fonctionnalisation.

**Patentansprüche**

1.  Verfahren zur Beobachtung einer Probe (EO), umfassend die Schritte bestehend aus:

    a) Bereitstellen eines Probenträgers (SAC), umfassend ein transparentes Substrat (ST), auf dem mindestens eine Schicht (CA) aufgebracht ist, die als Kontrastverstärkungsschicht bezeichnet wird, die einen komplexen Brechungskoeffizienten mit einem imaginären Teil $\kappa$ größer oder gleich 0,001 und vorzugsweise 0,01 aufweist;
    b) Anordnen der zu beobachtenden Probe auf der Kontrastverstärkungsschicht,
    c) Richten eines räumlich inkohärenten Lichtstrahls (F) auf die Probe durch das Substrat, der so fokussiert ist, dass er einen Lichtkegel bildet, der einen Öffnungshalbwinkel 8 größer oder gleich 20° aufweist; und
    d) Beobachten der Probe durch ein Objektiv (LO) und das Substrat; **dadurch gekennzeichnet, dass**
    die Kontrastverstärkungsschicht derart bemessen ist, dass ihre Dicke oder ihre lokale Dicke in einem bestimmten Bereich für mindestens eine Lichtwellenlänge den Kontrast, der in dem Lichtkegel integriert ist, mit dem eine Referenzprobe beobachtet werden soll, die aus einem transparenten Material von einer nanometrischen Dicke besteht, optimiert,
    und dadurch, dass
    der Lichtstrahl nicht polarisiert ist.

2.  Verfahren zur Beobachtung einer Probe nach Anspruch 1, wobei die Kontrastverstärkungsschicht metallisch ist.

3.  Verfahren zur Beobachtung einer Probe nach einem der vorhergehenden Ansprüche, wobei der Öffnungshalbwinkel des Lichtkegels zwischen 20° und 75°, vorzugsweise zwischen 30° und 70° und noch stärker bevorzugt zwischen 40° und 65° liegt, wobei die Achse des Kegels senkrecht zum Substrat steht.

4.  Verfahren zur Beobachtung einer Probe nach einem der vorhergehenden Ansprüche, wobei das Objektiv gleichzeitig zum Beleuchten und Beobachten der Probe verwendet wird.

5.  Verfahren zur Beobachtung einer Probe nach einem der vorhergehenden Ansprüche, wobei die Kontrastverstärkungsschicht und die Probe während des Schrittes d) in Wasser oder in eine wässrige Lösung eingetaucht werden.

6.  Verfahren zur Beobachtung einer Probe nach einem der vorhergehenden Ansprüche, wobei die Kontrastverstärkungsschicht einen Dickegradienten aufweist.

7.  Verfahren zur Beobachtung einer Probe nach einem der vorhergehenden Ansprüche, umfassend einen vorausgehenden Schritt der Bemessung der Kontrastverstärkungsschicht, umfassend:

    - Berechnen einer Oberfläche, die den Kontrast darstellt, mit dem die Referenzprobe beobachtet werden soll, in Abhängigkeit von der Dicke der Schicht, normalisiert in Bezug auf eine Lichtwellenlänge, und dem Öffnungshalbwinkel $\theta$ des Lichtstrahls;
    - Identifizieren eines Scheitels oder Talweges der Oberfläche, die eine Ausrichtung aufweist, die zur Achse, die den Öffnungshalbwinkel $\theta$ darstellt, in etwa parallel ist;
    - Identifizieren eines Wertes oder eines Wertebereichs der Dicke der Schicht entsprechend dem Scheitel oder Talweg;

    wobei die Dicke oder eine lokale Dicke der Kontrastverstärkungsschicht gleich dem so identifizierten Wert oder innerhalb des so identifizierten Wertebereichs gewählt wird.

8.  Verfahren zur Beobachtung einer Probe nach einem der vorhergehenden Ansprüche, wobei der Probenträger oberhalb der Kontrastverstärkungsschicht eine Funktionalisierungsschicht (CF) aufweist, die dafür geeignet ist, mindestens eine chemische oder biologische Spezies (ECO) zu fixieren, wobei das Verfahren auch einen Schritt des Inkontaktbringens der Funktionalisierungsschicht mit einer Lösung der zu fixierenden chemischen oder biologischen Spezies aufweist, wodurch die chemische oder biologische Spezies eine Schicht (CE) oberhalb der Funktionalisierungsschicht bildet, die die zu beobachtende Probe bildet, vorzugsweise wobei die oder mindestens eine chemische oder biologische Spezies absorbierend ist, die einen komplexen Brechungskoeffizienten mit einem imaginären Teil $\kappa$ größer oder gleich 0,0001 aufweist, oder streuend ist, und vorzugsweise wobei die Funktionalisierungsschicht in Form einer Vielzahl von Pads vorliegt, die dafür geeignet sind, die verschiedenen chemischen oder biologischen Spezies zu fixieren, wobei die Kontrastschicht optional nur den Pads entsprechend aufgebracht ist und der Träger optional außer den Pads eine Passivierungsschicht umfasst, die das Fixieren aller in der Lösung enthaltenen chemischen oder biologischen Spezies verhindert.

9. Verfahren zum Erfassen oder Dosieren mindestens einer chemischen oder biologischen Spezies, umfassend die Schritte bestehend aus:

a) Bereitstellen eines transparenten Substrats (ST), auf dem mindestens eine Funktionalisierungsschicht (CF) aufgebracht ist, die dafür geeignet ist, mindestens eine chemische oder biologische Spezies zu fixieren;
b) Platzieren der Funktionalisierungsschicht in Kontakt mit mindestens einer Lösung, die eine chemische oder biologische Spezies enthält, die durch metallische Nanopartikel oder einen absorbierenden oder streuenden Marker markiert ist, wobei die Spezies dafür geeignet ist, sich auf der Funktionalisierungsschicht entweder direkt oder mittels einer oder mehrerer anderer chemischer oder biologischer Spezies zu fixieren, wodurch die Partikel eine kontinuierliche oder diskontinuierliche, absorbierende oder streuende metallische Schicht (CM) bilden;
c) Richten, auf die Einheit, die aus mindestens der Funktionalisierungsschicht und der absorbierenden oder streuenden metallischen Schicht gebildet ist, durch das Substrat eines räumlich inkohärenten Lichtstrahls (F), der so fokussiert ist, dass er einen Lichtkegel bildet, der einen Öffnungshalbwinkel θ größer oder gleich 20° aufweist; und
d) Beobachten der Einheit durch ein Objektiv (LO) und durch das Substrat,

**dadurch gekennzeichnet, dass** der Lichtstrahl nicht polarisiert ist.

10. Verfahren zum Erfassen oder Dosieren mindestens einer chemischen oder biologischen Spezies nach Anspruch 9, wobei während des Schrittes b) die Funktionalisierungsschicht in Kontakt mit einer Lösung (S) platziert wird, die die chemische oder biologische, zu erfassende oder dosierende Spezies (ECO) enthält, die durch metallische Nanopartikel (NPM) oder einen absorbierenden oder streuenden Marker markiert ist, wobei die Spezies dafür geeignet ist, sich auf der Funktionalisierungsschicht zu fixieren, um die kontinuierliche oder diskontinuierliche metallische Schicht (CM) zu bilden.

11. Verfahren zum Erfassen oder Dosieren mindestens einer chemischen oder biologischen Spezies nach Anspruch 9, wobei Schritt b) die folgenden Unterschritte umfasst, bestehend aus:

b1) Platzieren der Funktionalisierungsschicht in Kontakt mit einer ersten Lösung (S1), die die zu erfassende oder dosierende chemische oder biologische Spezies enthält, so dass eine als Zwischenschicht (CI) bezeichnete Schicht gebildet wird; und
b2) Platzieren der Zwischenschicht in Kontakt mit einer zweiten Lösung (S2), die eine chemische oder biologische Spezies enthält, die als Hilfsspezies (ECA) bezeichnet wird, die durch metallische Nanopartikel oder einen absorbierenden oder streuenden Marker markiert ist und die dafür geeignet ist, sich auf der Zwischenschicht zu fixieren, um die kontinuierliche oder diskontinuierliche, absorbierende oder streuende metallische Schicht (CM) zu bilden.

12. Verfahren zum Erfassen oder Dosieren mindestens einer chemischen oder biologischen Spezies nach Anspruch 9, wobei Schritt b) die folgenden Unterschritte umfasst, bestehend aus:

b1) Platzieren der Funktionalisierungsschicht in Kontakt mit einer ersten Lösung (S1), die eine chemische oder biologische Spezies enthält, die als Zwischenspezies (ECI) bezeichnet wird, die durch metallische Nanopartikel oder einen absorbierenden oder streuenden Marker markiert ist und die dafür geeignet ist, sich auf der Funktionalisierungsschicht zu fixieren, um die kontinuierliche oder diskontinuierliche, absorbierende oder streuende metallische Schicht (CM) zu bilden; und
b2) Platzieren der Funktionalisierungsschicht und der absorbierenden oder streuenden metallischen Schicht in Kontakt mit einer zweiten Lösung (S2), die die zu erfassende oder dosierende chemische oder biologische Spezies enthält, die eine Affinität mit der Funktionalisierungsschicht aufweist, die größer als diejenige der Zwischenspezies ist, wodurch die Zwischenspezies verlagert und die absorbierende oder streuende metallische Schicht mindestens teilweise entfernt wird.

13. Verfahren zum Erfassen oder Dosieren mindestens einer chemischen oder biologischen Spezies nach Anspruch 9, wobei während des Schrittes b) die Funktionalisierungsschicht in Kontakt mit einer Lösung (S) platziert wird, die die zu dosierende chemische oder biologische Spezies ebenso wie die konkurrierende chemische oder biologische Spezies (ECC) enthält, wobei eine der beiden Spezies durch metallische Nanopartikel oder einen absorbierenden oder streuenden Marker markiert ist, wodurch eine kontinuierliche oder diskontinuierliche, absorbierende oder streuende metallische Schicht (CM) erhalten wird, deren effektive Dicke von dem Verhältnis zwischen der Konzentration der gleichzeitigen chemischen oder biologischen Spezies und derjenigen der zu dosierenden chemischen oder biologischen Spezies abhängt.

14. Verfahren zum Erfassen oder Dosieren nach einem der Ansprüche 9 bis 13, wobei der Öffnungshalbwinkel des Lichtkegels zwischen 20° und 75°, vorzugsweise zwischen 30° und 70° und noch stärker bevorzugt zwischen 40° und 65° liegt, wobei die Achse des Kegels senkrecht zum Substrat steht,

wobei vorzugsweise ein gleiches Objektiv zum Beleuchten und Beobachten der Probe verwendet wird, vorzugsweise wobei die Funktionalisierungsschicht in Form einer Vielzahl von Pads vorliegt, die auf der Oberfläche des Substrats angeordnet und dafür geeignet sind, die verschiedenen chemischen oder biologischen Spezies zu fixieren, wobei der Träger optional außer den Pads eine Passivierungsschicht umfasst, die das Fixieren aller in der Lösung enthaltenen chemischen oder biologischen Spezies verhindert, und vorzugsweise wobei eine Schicht, die als Kontrastverstärkungsschicht bezeichnet wird, die einen komplexen Brechungskoeffizienten mit einem imaginären Teil $\kappa$ größer oder gleich 0,001 und vorzugsweise 0,01 aufweist, zwischen dem Substrat und der Funktionalisierungsschicht eingeschoben ist.

## Claims

1. A method for observing a sample (EO) comprising the steps of:

   a) providing a sample support (SAC) comprising a transparent substrate (ST) on which there is deposited at least one layer (CA) which is referred to as a contrast amplification layer and which has a complex refraction index with an imaginary portion $\kappa$ which is greater than or equal to 0.001 and preferably 0.01;
   b) arranging the sample to be observed on the contrast amplification layer;
   c) directing onto the sample, through the substrate, a spatially incoherent light beam (F) which is focused in order to form an illumination cone which has an opening half-angle 8 which is greater than or equal to 20°; and
   d) observing the sample through an objective lens (LO) and the substrate; **characterised in that**:

      the contrast amplification layer is sized in such a manner that the thickness thereof, or the local thickness thereof in a predetermined region, optimises, for at least one illumination wavelength, the contrast integrated in the illumination cone with which a reference sample which is constituted by a transparent material of nanometric thickness would be observed,
      and **in that**:
      the light beam is non-polarised.

2. The method for observing a sample according to claim 1, wherein the contrast amplification layer is made of metal.

3. The method for observing a sample according to either of the preceding claims, wherein the opening half-angle of the illumination cone is between 20° and 75°, preferably between 30° and 70° and even more preferably between 40° and 65°, the axis of the cone being perpendicular to the substrate.

4. The method for observing a sample according to any one of the preceding claims, wherein the objective lens is used to illuminate and to observe the sample at the same time.

5. The method for observing a sample according to any one of the preceding claims, wherein the contrast amplification layer and the sample are immersed in water or an aqueous solution during step d).

6. The method for observing a sample according to any one of the preceding claims, wherein the contrast amplification layer has a thickness gradient.

7. The method for observing a sample according to any one of the preceding claims, comprising a preliminary step of dimensioning the contrast amplification layer, comprising:

- the calculation of a surface which represents the contrast with which the reference sample would be observed in accordance with the thickness of the layer, which is standardised with respect to an illumination wavelength, and the opening half-angle $\theta$ of the light beam;
- the identification of a peak or trough of the surface which has an orientation which is approximately parallel with the axis which represents the opening half-angle $\theta$;
- the identification of a value or range of values of the thickness of the layer which corresponds to the peak or trough;

the thickness or a local thickness of the contrast amplification layer being selected to be equal to the value or within the range of values identified in this manner.

8. The method for observing a sample according to any one of the preceding claims, wherein the sample support has, above the contrast amplification layer, a functionalisation layer (CF) which is capable of binding at least one chemical or biological species (ECO), the method also involving a step of placing the functionalisation layer in contact with a solution of the chemical or biological species to be bound, whereby the chemical or biological species forms a layer (CE) above the functionalisation layer, which constitutes the sample to be observed, preferably wherein the or at least one chemical or biological species is absorbent, having a complex refraction index with an imaginary portion $\kappa$ which is greater than or equal to 0.0001, or which is diffusive, and preferably wherein the functionalisation layer is in the form of a plurality of pads which are capable of binding different chemical or biological species, the contrast layer optionally being deposited only matching the pads and the support optionally comprising, in addition to the pads, a passivation layer which prevents any chemical or biological species contained in the solution from binding.

9. A method for detecting or metering at least one chemical or biological species, comprising the steps of:

    a) providing a transparent substrate (ST) on which there is deposited at least one functionalisation layer (CF) which is capable of binding at least one chemical or biological species;
    b) placing the functionalisation layer in contact with at least one solution which contains a chemical or biological species which is marked by metal nanoparticles or an absorbent or diffusive marker, the species being capable of binding to the functionalisation layer, either directly or via one or multiple other chemical or biological species, whereby the particles form a continuous or discontinuous absorbent or diffusive metal layer (CM);
    c) directing on the assembly constituted by at least said functionalisation layers and said absorbent or diffusive metal layers, through the substrate, a spatially incoherent light beam (F) which is focused in order to form an illumination cone which has an opening half-angle $\theta$ greater than or equal to 20°; and
    d) observing the assembly through an objective lens (LO) and through the substrate,

**characterised in that** the light beam is non-polarised.

10. The method for detecting or metering at least one chemical or biological species according to claim 9, wherein, during step b), the functionalisation layer is placed in contact with a solution (S) containing the chemical or biological species (ECO) which is intended to be detected or metered and which is marked by metal nanoparticles (NPM) or an absorbent or diffusive marker, the species being capable of being fixed to the functionalisation layer in order to form the continuous or discontinuous metal layer (CM).

11. The method for detecting or metering at least one chemical or biological species according to claim 9, wherein the step b) comprises the sub-steps of:

    b1) placing the functionalisation layer in contact with a first solution (S1) containing the chemical or biological species to be detected or metered in order to form a so-called intermediate layer (CI); and
    b2) placing the intermediate layer in contact with a second solution (S2) which contains a so-called auxiliary chemical or biological species (ECA) which is marked by metal nanoparticles or an absorbent or diffusive marker and which is capable of binding to the intermediate layer in order to form the absorbent or diffusive continuous or discontinuous metal layer (CM).

12. The method for detecting or metering at least one chemical or biological species according to claim 9, wherein the step b) comprises the sub-steps of:

    b1) placing the functionalisation layer in contact with a first solution (S1) which contains a chemical or biological species, which is referred to as an intermediate species (ECI) and which is marked by metal nanoparticles or an absorbent or diffusive marker and which is capable of binding to the functionalisation layer in order to form the absorbent or diffusive continuous or discontinuous metal layer (CM); and
b2) placing the functionalisation layer and the absorbent or diffusive metal layer in contact with a second solution (S2) which contains the chemical or biological species to be detected or metered, which has an affinity with the functionalisation layer which is greater than that of the intermediate species, whereby the intermediate species is displaced and the absorbent or diffusive metal layer is at least partially eliminated.

13. The method for detecting or metering at least one chemical or biological species according to claim 9, wherein, during step b), the functionalisation layer is placed into contact with a solution (S) containing the chemical or biological species to be metered and the competing chemical or biological species (ECC), one of the two species being marked by metal nanoparticles or an absorbent or diffusive marker, whereby an absorbent or diffusive continuous or discontinuous metal layer (CM) is obtained, the effective thickness of which is dependent on the relationship between the concentration of the chemical or biological species and that of the chemical or biological species to be metered.

14. The method for detecting or metering according to any one of claims 9 to 13, wherein the opening half-angle of the illumination cone is between 20° and 75°, preferably between 30° and 70° and even more preferably between 40° and 65°, the axis of the cone being perpendicular to the substrate,

preferably wherein the same objective lens is used to illuminate and to observe the sample,
preferably wherein the functionalisation layer is in the form of a plurality of pads which are arranged on the surface of the substrate and which are capable of binding different chemical or biological species, the support optionally comprising, in addition to the pads, a passivation layer which prevents any chemical or biological species contained in the solution from being bound, and preferably wherein a so-called contrast amplification layer which has a complex refraction index with an imaginary portion $\kappa$ which is greater than or equal to 0.001 and preferably 0.01 is interposed between the substrate and the functionalisation layer.

*Fig. 1*

*Fig. 2*

Fig. 3

Fig. 5

Fig. 4 - 1

[n₁; k₁] = [0,8; 2,5]

Fig. 4 - 2

[n₁; k₁] = [1; 0,5]

Fig. 4 - 3

Fig. 4 - 4

$[n_1; \ k_1] = \ [1; \ 2,0]$

*Fig. 4 - 5*

$[n_1; \ k_1] = \ [1; \ 2,5]$

*Fig. 4 - 6*

Fig. 4 - 7

Fig. 4 - 8

Fig. 4 - 9

Fig. 4 - 10

Fig. 4 - 11

Fig. 4 - 12

$[n_1; \ k_1] = [1{,}44; \ 0{,}25]$

*Fig. 4 - 13*

$[n_1; \ k_1] = [1{,}44; \ 0{,}2]$

*Fig. 4 - 14*

Fig. 4 - 15

Fig. 4 - 16

*Fig. 4 - 17*

$[n_1; \ k_1] = [1,7; \ 2,5]$

*Fig. 4 - 18*

$[n_1; \ k_1] = [1,75; \ 1,05]$

Fig. 4 - 19

$[n_1; \ k_1] = [1,6; \ 0.8]$

Fig. 4 - 20

$[n_1; \ k_1] = [1,65; \ 0,8]$

Fig. 4 - 21

$[n_1; \ k_1] = [1,7; \ 0,8]$

Fig. 4 - 22

$[n_1; \ k_1] = [1,7; \ 1]$

Fig. 4 - 23

$[n_1; \ k_1] = [1,8; \ 0,5]$

Fig. 4 - 24

$[n_1; \ k_1] = [1,8; \ 1]$

Fig. 4 - 25

$[n_1; k_1] = [1,9; 1,435]$

Fig. 4 - 26

$[n_1; k_1] = [1,9; 2,5]$

*Fig. 4 - 27* $[n_1; \ k_1] = [2; \ 1]$

$[n_1; \ k_1] = [2; \ 1,2]$

*Fig. 4 - 28*

Fig. 4 - 29

$[n_1; \ k_1] = [2; \ 1,3]$

Fig. 4 - 30

$[n_1; \ k_1] = [2; \ 1,4]$

Fig. 4 - 31

Fig. 4 - 32

$[n_1; \ k_1] = [2; \ 1,45]$

*Fig. 4 - 33*

$[n_1; \ k_1] = [2; \ 1,50]$

*Fig. 4 - 34*

Fig. 4 - 35

$[n_1; \ k_1] = [2; \ 1,70]$

Fig. 4 - 36

$[n_1; \ k_1] = [2; \ 2]$

[n₁; k₁] = [2; 2,5]

*Fig. 4 - 37*

[n₁; k₁] = [2; 3,5]

*Fig. 4 - 38*

$[n_1; \ k_1] = [2,1; \ 2,5]$

*Fig. 4 - 39*

$[n_1; \ k_1] = [2,1; \ 1,435]$

*Fig. 4 - 40*

$[n_1;\ k_1] = [2,2;\ 1,45]$

*Fig. 4 - 41*

$[n_1;\ k_1] = [2,2;\ 1,46]$

*Fig. 4 - 42*

$[n_1; \ k_1] = [2,2; \ 1,50]$

*Fig. 4 - 43*

$[n_1; \ k_1] = [2,2; \ 1,60]$

*Fig. 4 - 44*

$[n_1; \ k_1] = [2,2; \ 1,70]$

Fig. 4 - 45

$[n_1; \ k_1] = [2,2; \ 1,80]$

Fig. 4 - 46

$[n_1; \ k_1] = [3; \ 2{,}50]$

*Fig. 4 - 47*

$[n_1; \ k_1] = [3; \ 2{,}60]$

*Fig. 4 - 48*

$[n_1; \ k_1] = [3; \ 2{,}70]$

*Fig. 4 - 49*

$[n_1; \ k_1] = [4; \ 3{,}6]$

*Fig. 4 - 50*

$[n_1; \ k_1] = [4; \ 3,5]$

*Fig. 4 - 51*

$[n_1; \ k_1] = [4; \ 3,4]$

*Fig. 4 - 52*

Fig. 4 - 53

Fig. 4 - 54

$[n_1; \ k_1] = [6,7; \ 6,5]$

*Fig. 4 - 55*

$[n_1; \ k_1] = [7; \ 6,5]$

*Fig. 4 - 56*

Fig. 6

Fig. 7

Fig. 8

Fig. 9

Fig. 10

Fig. 11

Fig. 12

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004001399 A
- WO 0250513 A
- FR 2872910 A1

**Littérature non-brevet citée dans la description**

- **M. E. STEWART et al.** Nanostructured Plasmonic Sensors. *Chem. Rev.,* 2008, vol. 108, 494-521
- **S. A. MEYER et al.** Combining Surface Plasmon Resonance (SPR) Spectroscopy with Surface-Enhanced Raman Scattering (SERS). *Anal. Chem.,* 2011, vol. 83, 2337-2344
- **CHARLES T. CAMPBELL ; GIBUM KIM.** SPR microscopy and its applications to high-throughput analyses of biomolecular binding events and their kinetics. *Biomaterials,* 2007, vol. 28, 2380-2392
- **O. AUSSERRÉ ; M. P. VALIGNAT.** Surface enhanced ellipsometric contrast (SEEC) basic theory and $\lambda/4$ multilayered solutions. *Optics Express,* 25 Juin 2007, vol. 15 (13